# EUROPEAN PATENT SPECIFICATION

(11) **EP 3 936 782 B1**
(45) Date of publication and mention of the grant of the patent: **20.08.2025**
(21) Application number: 20765766.9
(22) Date of filing: 02.03.2020
(51) Int. Cl.: F24F 11/67, F24F 11/00, F24F 11/77, F24F 1/037, A61L 2/07, F24F 6/02, F24F 110/10, F24F 110/20, F24F 6/00, F24F 11/65, F24F 11/86, F24F 1/0087, A61L 2/04, A61L 9/04

(54) **INDOOR UNIT OF AIR CONDITIONER AND CONTROL METHOD THEREFOR**
INNENRAUMEINHEIT EINER KLIMAANLAGE UND STEUERVERFAHREN DAFÜR
UNITÉ INTÉRIEURE DE CLIMATISEUR ET SON PROCÉDÉ DE COMMANDE

(30) Priority: 04.03.2019 KR 20190024910
(43) Date of publication of application: 12.01.2022
(73) Proprietor: LG Electronics Inc., SEOUL 07336 (KR)
(72) Inventor: KWON, Mingu, Seoul 08592 (KR); YEO, Kyungmok, Seoul 08592 (KR); LEE, Young Gu, Seoul 08592 (KR); SONG, Jaeyong, Seoul 08592 (KR)
(74) Representative: Vossius & Partner Patentanwälte Rechtsanwälte mbB
(86) International application number: PCT/KR2020/002975
(87) International publication number: WO 2020/180075

(56) References cited:
- WO-A1-2019/008694
- WO-A1-2019/033700
- JP-A- 2002 089 878
- JP-A- 2002 089 878
- KR-A- 19990 005 840
- KR-A- 20180 127 223
- KR-A- 20180 127 223
- KR-A- 20190 019 737
- KR-A- 20190 019 737
- KR-A- 970 007 140
- KR-B1- 0 144 064
- KR-B1- 100 144 064

## Description

The present invention relates to an indoor unit of an air conditioner and a control method thereof, and more particularly, to an indoor unit of an air conditioner capable of providing humidified air to an indoor space and a control method thereof.

A split-type air conditioner may include an indoor unit disposed in an indoor space and an outdoor unit disposed in an outdoor space and may cool, heat, or dehumidify indoor air through refrigerants circulating between the indoor unit and the outdoor unit.

Examples of indoor unit of the split-type air conditioner include a stand-type indoor unit vertically disposed on the indoor floor, a wall-mounted indoor unit mounted on the indoor wall, and a ceiling-type indoor unit mounted on the indoor ceiling according to installation forms.

The stand-type indoor unit in related art may dehumidify indoor air during cooling, but may not humidify indoor air during heating.

Korean Patent Publication No. 10-2013-0109738 (referred to as Related art document 1) discloses a stand-type indoor unit including a humidification device capable of providing humidification. According to the related art document 1, the stand-type indoor unit includes a humidification device inside a main body defining an appearance of the indoor unit. In addition, according to the related art document 1, the humidification device stores water in a drain fan in a water tank, wets an absorbing member with the stored water, and naturally evaporates the water absorbed by the absorbing member.

According to the related art document 1, the humidification device does not use clean water, but uses condensed water flowing down from a heat exchanger. The water stored in the water tank may contain a large amount of foreign substances separated from a surface of the heat exchanger, and there is a problem in that it is highly probable that mold or virus propagates from the foreign substances.

According to the related art document 1, as the humidification device evaporates water inside the main body, there is a problem in that the evaporated water may be adhered to components or the inner wall of the main body, thereby causing the propagation of the mold or the virus in the main body. In addition, according to the related art document 1, there is a problem in that the humidification device evaporates the water inside the main body, and even if a blowing fan is operated, all the evaporated moisture is not discharged into an indoor space by the blowing fan, and if a temperature of an indoor heat exchanger is low, the moisture is reattached to the surface of the indoor heat exchanger.

When an indoor temperature is low, humidity of the indoor air is low. In this case, the humidification is generally needed during heating. However, there is a problem in that the humidification device in the related art document 1 may provide humidification only during cooling because the humidification device provides the humidification using the condensed water of the indoor heat exchanger, but may not provide humidification because condensed water is not generated during heating.

WO 2019/033700 (A1) relates to an air processing module and an air conditioner using the air processing module.

The invention is specified by the independent claims. Preferred embodiments are defined in the dependent claims.

The present invention thereafter referred to as disclosure provides an indoor unit of an air conditioner that may be operated in at least one of a cooling mode, a heating mode, an air cleaning mode, or a humidification mode.

The present disclosure also provides an indoor unit of an air conditioner to heat stored or supplied water to sterilize the water, change the sterilized water to steam to provide humidification.

The present disclosure further provides an indoor unit of an air conditioner having an independent flow path structure that may provide filtered air to a steam generator, to generate steam using the filtered air, and provide the humidified air generated by the steam generator to a discharge outlet through the independent air flow.

The present disclosure further provides an indoor unit of an air conditioner to provide humidified air using clean water by setting a use time period of stored water and draining the water.

The present disclosure further provides an indoor unit of an air conditioner to sterilize a flow path through which humidified air flows, after the humidification.

The present disclosure provides an indoor unit of an air conditioner to set an operation based on a detected amount of water and quickly provide humidified air based on a preliminary operation.

The present disclosure further provides a comfortable environment in which a temperature and humidity are controlled without unnecessary manipulation.

The problems of the present disclosure are not limited to the problems mentioned above, and other problems, which are not mentioned, will be clearly understood by those skilled in the art from the following description.

According to the present disclosure, an indoor unit includes a humidification assembly to generate steam, mix the generated steam with filtered air to generate humidified air, and spray the generated humidified air at a discharge outlet through an independent flow path to provide the humidified air to an indoor space and may be operated in at least one of a cooling mode, a heating mode, an air cleaning mode, or a humidification mode.

According to the present disclosure, a steam generator changes stored water to steam to generate humidified air, a humidification fan supplies the filtered air that has passed through a filter assembly to the steam generator, and a steam guide guides the humidified air discharged from the steam generator to the discharge outlet through an independent flow path to directly discharge the generated humidified air to an indoor space.

According to the present disclosure, a humidification module performs a water boiling operation as preliminary humidification to generate humidified air in a short time, performs a humidification operation to provide humidified air, and after the humidification operation, performs a water cooling operation step by step to remove residual moisture.

According to the present disclosure, the humidification module performs a steam sterilization operation to sterilize a flow path through humidified air flows after the humidification operation.

According to the present disclosure, an indoor unit automatically switches an operation mode thereof based on detected temperature and humidity while discharging heat-exchanged air to discharge air with adjusted temperature and humidity to an indoor space.

According to the present disclosure, the humidification module may count a use time period of the stored water to automatically drain the water that remains for a predetermined time period or more.

According to the present disclosure, an air conditioner includes a fan having a short-distance fan assembly and a long-distance fan assembly and configured to discharge air; a cabinet assembly defining a suction inlet and a discharge outlet; a water tank disposed in the cabinet assembly and configured to store water; a steam generator disposed in the cabinet assembly, configured to receive the water stored in the water tank, change the stored water to steam to generate humidified air; a humidification fan coupled to the steam generator and configured to supply filtered air to the steam generator; and a controller configured to: control an operation thereof in a smart care mode operating the steam generator, during heating operation by controlling a compressor and the fan, to discharge the humidified air together with heat-exchanged air to adjust a temperature and humidity constantly.

According to the present disclosure, a method for controlling an indoor unit includes performing a heating operation in a high speed mode after a smart care mode is set; discharging heat-exchanged air by operating both a short-distance fan assembly and a long-distance fan assembly; switching the high speed mode to a comfortable mode based on an indoor temperature reaching to a desired temperature; detecting the indoor temperature and indoor humidity while operating in the comfortable mode; performing a humidification operation by operating a steam generator to maintain the indoor humidity within a specified humidity range; and releasing the comfortable mode and switching to the high speed mode based on a temperature difference between the indoor temperature and the desired temperature reaching to a predetermined value.

An indoor unit of an air conditioner according to the present disclosure has one or more of the following effects.

There is an advantage in that the indoor unit of the present disclosure may be operated in at least one of a cooling mode, a heating mode, an air cleaning mode, or a humidification mode and may be used for four seasons.

There is an advantage in that the indoor unit of the present disclosure sterilizes water by heating stored or supplied water, changes the sterilized water to steam, and provides humidified air by mixing sterilized steam and filtered air, thereby reducing a possibility of contamination of the generated humidified air and obtaining reliability of the humidified air.

According to the present disclosure, humidified air may be quickly generated by boiling water before generating humidified air.

According to the present disclosure, there is an advantage in that propagation of mold or virus may be minimized as the humidified air is supplied with the sterilized steam and the filtered air.

According to the present disclosure, there is an advantage in that the propagation of the mold may be minimized by sterilizing a flow path through which humidified air flows by steam sterilization, after the humidification operation.

According to the present disclosure, there is an advantage in that the propagation of the mold due to residual moisture may be minimized by drying the steam generator and a humidification guide after the humidification.

According to the present disclosure, energy may be saved by preventing frequent on and off of the air conditioner.

According to the present disclosure, the air conditioner controls the discharged air and the humidified air by automatically switching an operation mode thereof based on a temperature and humidity, thereby effectively supplying the air with the adjusted temperature and humidity.

According to the present disclosure, a use time period of the stored water may be counted to automatically drain the water to block water storage for a predetermined time period or more, thereby generating clean humidified air and minimizing a possibility of contamination of the humidified air.

According to the present disclosure, as the humidified air is diffused together with wind discharged from each of side discharge outlets, the humidified air may flow to a remote place.

According to the present disclosure, there is an advantage in that the humidified air is discharged to a space in front of the air discharged from the side discharge outlet and the humidified air may be effectively diffused to the indoor space based on the flow of the discharged air.

### [Description of Drawings]

FIG. 1 is a perspective view of an indoor unit of an air conditioner according to an embodiment of the present disclosure.
FIG. 2 is a perspective view of an indoor unit from which a door assembly in FIG. 1 is removed.
FIG. 3 is a front view of an interior of a lower cabinet in FIG. 2.
FIG. 4 is a cross-sectional view of a humidification assembly and a water tank in FIG. 3.
FIG. 5 is a schematic block diagram of components of an air conditioner according to an embodiment of the present disclosure.
FIG. 6 is a block diagram of components of a processor for controlling an air conditioner according to an embodiment of the present disclosure.
FIG. 7 is a schematic block diagram of components of a humidification module of an air conditioner according to an embodiment of the present disclosure.
FIG. 8 is a flowchart of an operation of providing air humidified by an air conditioner according to an embodiment of the present disclosure.
FIG. 9 show airflows according to operation steps of an air conditioner according to an embodiment of the present disclosure.
FIG. 10 is a flowchart of a water management method by an air conditioner according to an embodiment of the present disclosure.
FIG. 11 is a flowchart of a method for boiling water by an air conditioner according to an embodiment of the present disclosure.
FIG. 12 is a flowchart of a method for performing a humidification operation by an air conditioner according to an embodiment of the present disclosure.
FIG. 13 is a flowchart of a method for performing a water cooling operation by an air conditioner according to an embodiment of the present disclosure.
FIG. 14 is a flowchart of a method for automatically controlling a temperature and humidity by an air conditioner according to an embodiment of the present disclosure.

### [Best Mode]

The above-mentioned objects, features, and advantages of the present disclosure are described below in detail with reference to accompanying drawings. Therefore, the skilled person in the art to which the present disclosure pertains may easily embody the technical idea of the present disclosure. In the description of the present disclosure, a detailed description of a well-known technology relating to the present disclosure may be omitted if it unnecessarily obscures the gist of the present disclosure. Hereinafter, preferred embodiments of the present disclosure are described in detail with reference to the accompanying drawings. In the drawings, same reference numerals are used to refer to same or similar components.

Terms such as first, second, and the like may be used herein to describe various elements of the present disclosure. These elements are not limited by these terms. These terms are intended to distinguish one element from another element. A first element may be a second element unless otherwise stated.

In this document, the terms "upper," "lower," "on," "under," or the like are used such that, where a first component is arranged at "an upper portion" or "a lower portion" of a second component, the first component may be arranged in contact with the upper surface or the lower surface of the second component, or another component may be disposed between the first component and the second component. Similarly, where a first component is arranged on or under a second component, the first component may be arranged directly on or under (in contact with) the second component, or one or more other components may be disposed between the first component and the second component.

Further, the terms "connected," "coupled," or the like are used such that, where a first component is connected or coupled to a second component, the first component may be directly connected or able to be connected to the second component, or one or more additional components may be disposed between the first and second components, or the first and second components may be connected or coupled through one or more additional components.

Unless otherwise stated, each component may be singular or plural throughout the disclosure.

Singular expressions used in the present disclosure include plural expressions unless the context clearly displays otherwise. In the present disclosure, terms such as "including" or "comprising" should not be construed as necessarily including all of the various components, or various steps described in the present disclosure, and terms such as "including" or "comprising" should be construed as not including some elements or some steps or further including additional elements or steps.

In the present disclosure, unless otherwise stated, "A and/or B" means A, B, or A and B. Unless otherwise stated, "C to D" means "C or more and D or less".

Hereinafter, an indoor unit of an air conditioner and a controlling method thereof according to some embodiments of the present disclosure are described.

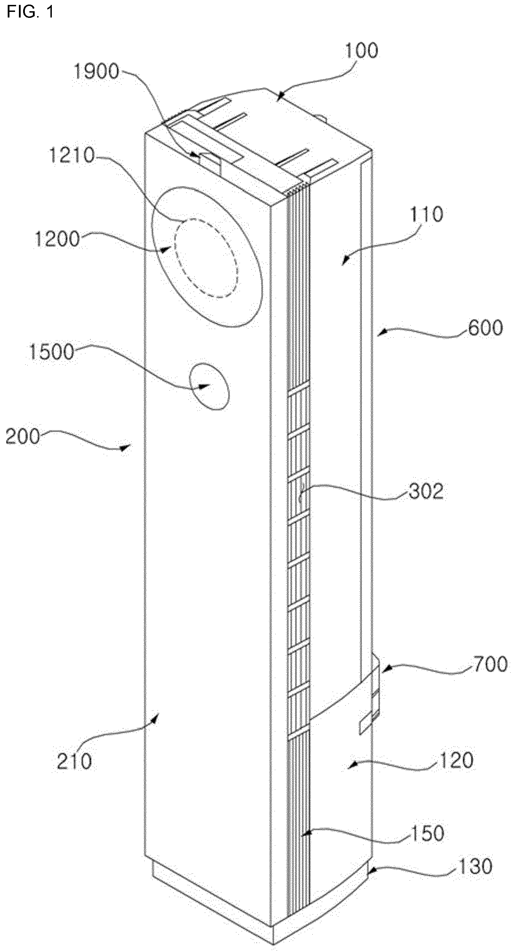

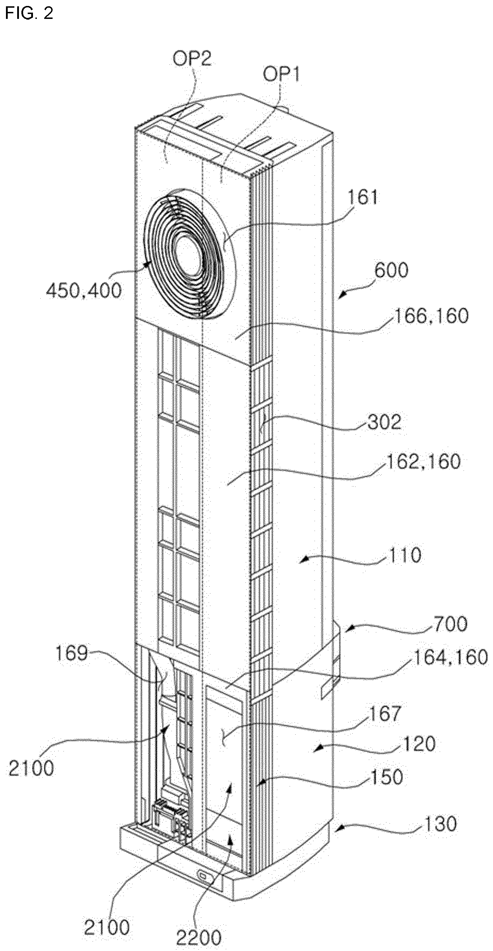

FIG. 1 is a perspective view of an indoor unit of an air conditioner according to an embodiment of the present disclosure. FIG. 2 is a perspective view of an indoor unit from which a door assembly in FIG. 1 is removed.

According to this embodiment, the air conditioner includes an indoor unit and an outdoor unit (not shown) connected to the indoor unit through a refrigerant pipe and circulates refrigerant.

The outdoor unit includes a compressor (not shown) to compress the refrigerant, an outdoor heat exchanger (not shown) to receive the refrigerant from the compressor and condense the refrigerant, an outdoor fan (not shown) to supply air to the outdoor heat exchanger, and an accumulator (not shown) to receive the refrigerant discharged by the indoor unit and provide only gas refrigerant to the compressor.

The outdoor unit may further include a four-way valve (not shown) to operate the indoor unit in a cooling mode or a heating mode. When the indoor unit is operated in the cooling mode, the indoor unit evaporates the refrigerant to cool indoor air. When the indoor unit is operated in the heating mode, the indoor unit condenses the refrigerant to heat the indoor air.

### <<Configuration of indoor unit>>

The indoor unit includes a cabinet assembly 100 defining an opening at a front surface thereof and a suction inlet 101 at a rear surface thereof, a door assembly 200 assembled to the cabinet assembly 100, to cover the front surface of the cabinet assembly 100, and open and close the front surface of the cabinet assembly 100, fan assemblies 300 and 400 disposed in an inner space (S) of the cabinet assembly 100 and to discharge air in the inner space (S) to an indoor space, a heat exchange assembly 500 disposed between the fan assemblies 300, 400 and the cabinet assembly 100 and to heat-exchange the suctioned indoor air with refrigerant, a humidification assembly 2000 disposed in the cabinet assembly 100 and to provide moisture to an indoor space, a filter assembly 600 disposed on a rear surface of the cabinet assembly 100 and to filter air flowing to the suction inlet 101, and a moving cleaner 700 vertically moving along the filter assembly 600 and to separate foreign substances in the filter assembly 600 and collect the foreign substances.

The indoor unit includes the suction inlet 101 defined on the rear surface of the cabinet assembly 100, side discharge outlets 301 and 302 defined at side surfaces of the cabinet assembly 100, and a front discharge outlet 201 defined on a front surface of the cabinet assembly 100.

The suction inlet 101 is defined on the rear surface of the cabinet assembly 100.

The side discharge outlets 301 and 302 are defined on the left side and the right side of the cabinet assembly 100, respectively. In this embodiment, when viewed from the front of the cabinet assembly 100, the side discharge outlet defined on the left side thereof is referred to as the first side discharge outlet 301 and the side discharge outlet defined on the right side thereof is referred to as the second side discharge outlet 302.

The door assembly 200 defines the front discharge outlet 201 and further includes a door cover assembly 1200 to automatically open and close the front discharge outlet 201.

The door cover assembly 1200 may be moved downward along the door assembly 200 after opening the front discharge outlet 201. The door cover assembly 1200 may be movable vertically with respect to the door assembly 200.

After the door cover assembly 1200 moves downward, the long-distance fan assembly 400 may move forward through the door assembly 200.

The fan assemblies 300 and 400 include the short-distance fan assembly 300 and the long-distance fan assembly 400. The heat exchange assembly 500 is disposed behind each of the shot-distance fan assembly 300 and the long-distance fan assembly 400.

The heat exchange assembly 500 is disposed in the cabinet assembly 100, is disposed inside the suction inlet 101, covers the suction inlet 101, and is vertically disposed.

The short-distance fan assembly 300 and the long-distance fan assembly 400 are each disposed in front of the heat exchange assembly 500. The air suctioned via the suction inlet 101 passes through the heat exchange assembly 500 and flows to each of the short-distance fan assembly 300 and the long-distance fan assembly 400.

The heat exchange assembly 500 is manufactured to have a length corresponding to a height of the short-distance fan assembly 300 and the long-distance fan assembly 400.

The short-distance fan assembly 300 and the long-distance fan assembly 400 may be vertically stacked. In this embodiment, the long-distance fan assembly 400 is disposed on the short-distance fan assembly 300. As the long-distance fan assembly 400 is disposed thereon to flow the discharged air to a remote place in the indoor space.

The short-distance fan assembly 300 discharges air in a lateral direction of the cabinet assembly 100. The short-distance fan assembly 300 may provide an indirect airflow to a user. The short-distance fan assembly 300 simultaneously discharges air to the left side and the right side of the cabinet assembly 100.

The long-distance fan assembly 400 is disposed on the short-distance fan assembly 300 and is disposed in the cabinet assembly 100 at an upper portion thereof.

The long-distance fan assembly 400 discharges the air in a forward direction of the cabinet assembly 100. The long-distance fan assembly 400 provides direct airflow to the user. In addition, the long-distance fan assembly 300 discharges the air to the remote place in the indoor space to improve indoor air circulation.

In this embodiment, the long-distance fan assembly 400 is exposed to the user only when the long-distance fan assembly 400 is operated. When the long-distance fan assembly 400 is operated, the long-distance fan assembly 400 passes through the door assembly 200 and is exposed to the user. When the long-distance fan assembly 400 is not operated, the long-distance fan assembly 400 is concealed inside the cabinet assembly 100.

In particular, the long-distance fan assembly 400 may control an air discharging direction. The long-distance fan assembly 400 may discharge the air upward, downward, leftward, rightward, or diagonally with respect to the front of the cabinet assembly 100.

The door assembly 200 is disposed on a front surface of the cabinet assembly 100 and is assembled to the cabinet assembly 100.

The door assembly 200 may horizontally slide relative to the cabinet assembly 200 and may expose, to outside, a portion of a front surface of the cabinet assembly 200.

The door assembly 200 is moved in at least one of a leftward direction or a rightward direction to open an inner space (S). In addition, the door assembly 200 is moved in the at least one of the leftward direction or the rightward direction to open only a portion of the inner space (S).

In this embodiment, the door assembly 200 is opened and closed with two steps.

A first opening and closing of the door assembly 200 refers to a partial opening in order for the humidification assembly 2000 to supply water and expose only an area of a water tank 2100 of the humidification assembly 2000.

A second opening and closing of the door assembly 200 refers to a full opening and is configured for installation and repair. The door assembly 200 has a door stopper structure to restrict the second opening and closing.

The filter assembly 600 is disposed on a rear surface of the cabinet assembly 100. The filter assembly 600 may be rotated to the side of the cabinet assembly 100 when the filter assembly 600 is disposed on the rear surface of the cabinet assembly 100. The user may separate only a filter from the filter assembly 600 moved to the side of the cabinet assembly 100.

In this embodiment, the filter assembly 600 includes two portions and each of the two portions thereof may be rotated leftward or rightward.

The moving cleaner 700 cleans the filter assembly 600. The moving cleaner 700 may vertically move to clean the filter assembly 600. The moving cleaner 700 may suction air while moving to separate foreign substances attached to the filter assembly 600, and the separated foreign substances are stored in the moving cleaner 700.

The moving cleaner 700 does not interfere with the filter assembly 600 when the filter assembly 600 rotates.

The humidification assembly 2000 provides moisture to the inner space (S) of the cabinet assembly 100 and the provided moisture may be discharged to the indoor space by the short-distance fan assembly. The humidification assembly 2000 includes a detachable water tank 2100.

In this embodiment, the humidification assembly 2000 is disposed at a lower portion thereof inside the cabinet assembly 100. A space in which the humidification assembly 2000 is disposed is partitioned from a space in which the heat exchange assembly 500 is disposed.

The humidification assembly 2000 performs humidification using air filtered by the filter assembly 600 and sterilized steam, thereby blocking harmful substances such as virus or mold from contact with the water tank.

### <<Configuration of cabinet assembly>>

The cabinet assembly 100 includes a base 130 disposed on the floor, a lower cabinet 120 disposed on the base 130, and an upper cabinet 110 disposed on the lower cabinet 120.

A short-distance fan assembly 300, a long-distance fan assembly 400, and a heat exchange assembly 500 are each disposed inside the upper cabinet 110.

A humidification assembly 2000 is disposed inside the lower cabinet 120.

A door assembly 200 is disposed at a front side of the cabinet assembly 100 and may slide horizontally relative to the cabinet assembly 100.

When the door assembly 200 is moved, a portion of a left side or a right side of the cabinet assembly 100 may be exposed to outside.

A discharge grill 150 is disposed at an edge of a front side of the upper cabinet 110. The discharge grill 340 is disposed behind the door assembly 200.

The discharge grill 150 may be manufactured to be integrated with the upper cabinet 110. In this embodiment, the discharge grill 150 is separately manufactured by injection molding and then is assembled to the upper cabinet 110.

In this embodiment, a cover 160 is disposed at a front side of the upper cabinet 110 and the lower cabinet 120 to block direction contact of the air inside the cabinet assembly 100 with the door assembly 200.

When cold air directly contacts the door assembly 200, there is a problem in that dew condensation may occur, thereby negatively affecting an electric circuit of the door assembly 200.

The cover 160 is disposed at the front side of the upper cabinet 110 and the lower cabinet 120 and may flow the air inside the cabinet assembly 100 only to a front discharge outlet 201 or side discharge outlets 301 and 302.

The cover 160 includes an upper cover 162 to cover a front surface of the upper cabinet 110, a lower cover 164 to cover a front surface of the lower cabinet 120, and a long-distance fan cover 166 to cover a front surface of the long-distance fan assembly 400.

At a time of first opening of the door assembly 200, the lower cover 164 defining a water tank opening 167 is only exposed to a user, and at a time of second opening of the door assembly 200, the open surface 169 is also exposed to the user.

The door assembly 200 slides in the horizontal direction by the operation of the door slide module 1300. A state in which the water tank opening 167 is entirely exposed based on the sliding movement of the door assembly 200 is referred to as "the first opening" and a state in which the open surface 169 is exposed is referred to as "the second opening".

At the time of first opening, the exposed front surface of the cabinet assembly 100 is referred to as "a first open surface (OP1)", and at the time of second opening, the exposed front surface of the cabinet assembly is referred to as "a second open surface (OP2)".

### <<Configuration of short-distance fan assembly>>

The short-distance fan assembly 300 discharges air in a lateral direction of the cabinet assembly 100. The short-distance fan assembly 300 provides an indirect air flow to a user.

The short-distance fan assembly 300 is disposed in front of the heat exchange assembly 500.

The short-distance fan assembly 300 includes a plurality of fans 310 that are vertically stacked. In this embodiment, the short-distance fan assembly 300 includes three fans 310 that are vertically stacked.

In this embodiment, the fan 310 uses a centrifugal mixed flow fan. The fan 310 suctions air in an axial direction and discharges the air in a circumferential direction.

### <<Configuration of long-distance fan assembly>>

The long-distance fan assembly 400 discharges air in a forward direction of the cabinet assembly 100. The long-distance fan assembly 400 provides a direct air flow to a user.

The long-distance fan assembly 400 is disposed in front of the heat exchange assembly 500. The long-distance fan assembly 400 is stacked on an upper surface of the short-distance fan assembly 300.

The long-distance fan assembly 400 discharges air through a front discharge outlet 201 of the door assembly 200. The long-distance fan assembly 400 provides a structure rotatable in an upward direction, a downward direction, a leftward direction, a rightward direction, or a diagonal direction. The long-distance fan assembly 400 may improve circulation of indoor air by discharging the air to a remote place in an indoor space.

The long-distance fan assembly 400 further includes a tilting assembly to freely rotate the discharge grill 450 relative to the fan housing assembly in all directions, such as the upward direction, the downward direction, the leftward direction, the rightward direction, and the diagonal direction.

### <<<Configuration of door assembly>>>

The door assembly 200 includes a front panel 210 defining a front discharge outlet 201, a door cover assembly 1200, a door slide module 1300, and a camera module 1900 to capture an image of an indoor space.

The front panel 210 defines the front discharge outlet 201 that is opened in a forward and rearward direction.

The display module 1500 is disposed on a rear surface of the front panel 210 and may provide visual information to a user through the front panel.

The display module 1500 is partially exposed through the front panel 210 and may provide the visual information to the user on the exposed display.

The door cover assembly 1200 opens and closes the front discharge outlet 201 of the door assembly 200.

The door cover assembly 1200 opens the front discharge outlet 201 to expand a movement path of a long-distance fan assembly 400. The long-distance fan assembly 400 may protrude to an outside of the door assembly 200 through the open front discharge outlet 201.

The door cover assembly 1200 is provided on the movement path of the long-distance fan assembly 400, and when the front discharge outlet 201 is opened, the door cover assembly 1200 is moved out of the movement path of the long-distance fan assembly 400.

In the first front opening, the long-distance fan assembly 400 is covered by the door cover 1210 and is not exposed to the user. In the first front opening, air inside a cabinet may be discharged into an indoor space through a gap between the door cover 1210 and the front panel 210.

In the first front opening, the long-distance fan assembly 400 is disposed behind the door cover 1210. In the first front opening, the door cover 1210 is disposed behind a front panel body 212.

In the second front opening, the door cover 1210 is disposed below each of the front discharge outlet 201 and the long-distance fan assembly 400. In the second front opening, the door cover 1210 is disposed behind the front panel body 212.

In the second front opening, the long-distance fan assembly 400 is exposed to the user through the front discharge outlet 201. In the second front opening, the long-distance fan assembly 400 is moved forward to protrude outward from the front discharge outlet 201 and may discharge air toward an indoor space when the long-distance fan assembly 400 protrudes outward from the front panel 210.

The door slide module 1300 moves a door assembly 200 in a horizontal direction of a cabinet assembly 100. The door slide module 1300 may reciprocate the door assembly 200 in the horizontal direction.

The door slide module 1300 is disposed in one of the door assembly 200 or the cabinet assembly 100 and slides by interference with the other one thereof.

The door detection sensor 207 detects a sliding movement distance of the door assembly 200. The position detection factor 208 is disposed on the door assembly 200.

The position detection factor 208 corresponds to the door detection sensor 207. The position detection factor 208 is disposed on a rear surface of the door assembly 200, and specifically, is disposed on a rear surface of a lower panel module 1120.

In this embodiment, a Hall sensor and a permanent magnet are used to detect a horizontal movement distance of the door assembly 200. The Hall sensor is used as the door detection sensor 207 and the permanent magnet is used as the position detection factor 208.

In another embodiment, a photo sensor is used as the door detection sensor and a rib disposed on the door assembly is used as the position detection factor. When the rib blocks an optical signal of the photo sensor, the horizontal movement distance of the door assembly may be determined.

### <<Configuration of camera module>>

The camera module 1900 is disposed in a door assembly 200 (e.g., an upper panel module 1110 in this embodiment) and is selectively operated. The camera module 1900 is exposed to an outside of the door assembly 200 only during operation thereof and is concealed inside the door assembly 200 when not in operation.

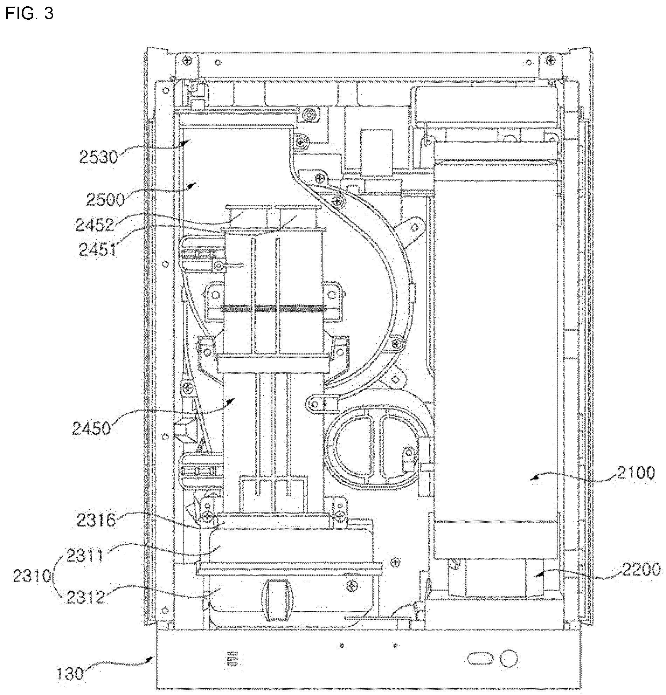

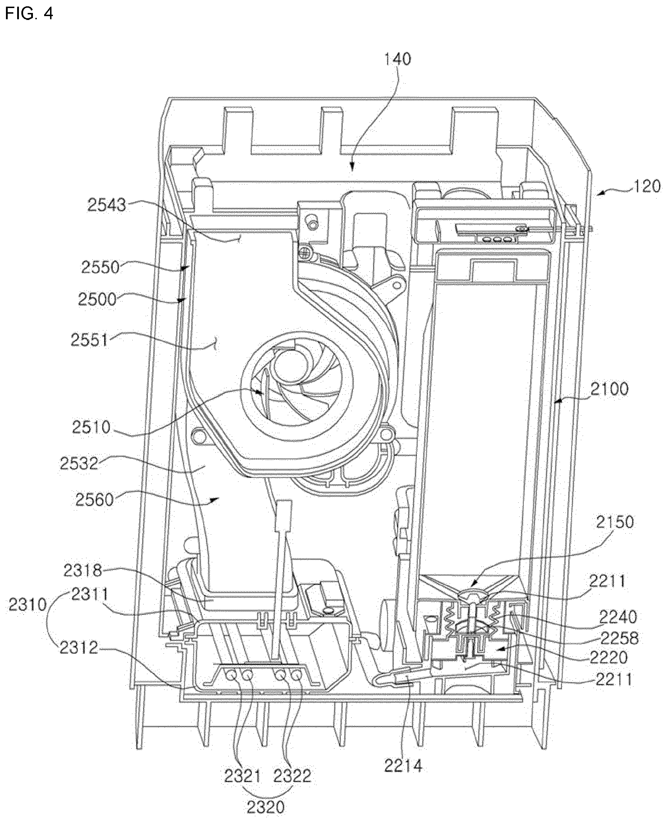

FIG. 3 is a front view of an interior of a lower cabinet in FIG. 2. FIG. 4 shows a humidification assembly and a water tank in FIG. 3.

### <<<Configuration of humidification assembly>>>

The humidification assembly 2000 may provide moisture to a discharge flow path of the fan assemblies 300 and 400 and the provided moisture may be discharged to an indoor space. The humidification assembly 2000 may be selectively operated based on a manipulation signal of a controller.

In this embodiment, the moisture provided by the humidification assembly 2000 may be directly supplied to side discharge outlets 301 and 302. The moisture supplied from the humidification assembly 2000 may be in an atomized state or in a steam state. In this embodiment, the humidification assembly 2000 changes water in a water tank 2100 to steam and supplies the steam to the discharge flow path.

In this embodiment, the humidification assembly 2000 is disposed at a lower portion of an inner side of the cabinet assembly 100, specifically, inside the lower cabinet 120.

The humidification assembly 2000 is disposed on a base 130 and is covered by the lower cabinet 120. A drain fan 140 is disposed on the humidification assembly 2000 and the steam generated by the humidification assembly 2000 directly flows to the side discharge outlets 301 and 302 through a steam guide (not shown). That is, a space where the humidification assembly 2000 is disposed is partitioned from a space inside the upper cabinet 110.

The humidification assembly 2000 includes a water tank 2100 disposed in the cabinet assembly 100 and to store water, a steam generator 2300 disposed in the cabinet assembly 100 and to receive water stored in the water tank 2100, change the water stored therein to steam to generate humidified air, a humidification fan 2500 disposed in the cabinet assembly 100, coupled to the steam generator 2300, and to supply air that is filtered through the filter assembly 600 to the steam generator 2300, and a steam guide 2400 disposed in the cabinet assembly 100 and to guide the humidified air generated by the steam generator 2300 to the side discharge outlets 301 and 302 of the cabinet assembly 100 via an independent flow path.

In addition, the humidification assembly includes a water supply assembly 2200 disposed in the cabinet assembly 100, to detachably support the water tank 2100 and supply the water in the water tank 2100 to the steam generator 2300, a tilting assembly disposed in the cabinet assembly 100 or the water supply assembly 2200 and to selectively tilt the water tank 2100 in a forward direction based on an electrical signal and return the water tank tilted in the forward direction to an original position thereof, and a drain assembly connected to the water supply assembly 2200 and the steam generator 2300 to drain water in the water supply assembly 2200 and the steam generator 2300 to outside.

### <<Configuration of water tank>>

The water tank 2100 is exposed to outside at a time of first opening of a door assembly 200 and is not exposed to outside when the door assembly 200 is not opened.

The door assembly 200 slides in a horizontal direction based on an operation of a door slide module 1300. A state in which a water tank opening 167 is entirely exposed by a sliding movement of the door assembly 200 is referred to as first opening and a state in which the open surface 169 is exposed is referred to as second opening.

In this embodiment, at least a portion of a front surface of the water tank 2100 is made of material that may see the water in the water tank 2100. The water tank 2100 is disposed on the first open surface (OP1), and more specifically, is disposed in the water tank opening 167. The water tank 2100 is inserted into a lower cabinet 120 through the water tank opening 167.

The water tank 2100 includes a tank lower body 2110 supported on a water supply assembly 2200, a tank middle body 2120 defining openings at a front side and a lower side thereof, coupled to an upper surface of the tank lower body 2110, having a lower surface closed by the tank lower body 2110, and to store water, a tank upper body 2130 defining a water tank opening 2101 at an upper side and a lower side thereof and coupled to an upper surface of the tank middle body 2120, a water tank handle 2140 rotatably assembled to the tank upper body 2130, and a water tank valve 2150 assembled to the tank lower body 2110 and to selectively supply water stored therein to the water supply assembly 2200.

The tank lower body 2110 provides a bottom of the water tank 2100. The tank lower body 2110 includes a valve hole 2111 that penetrates in a vertical direction and the water tank valve 2150 is assembled to the valve hole 2111. The valve hole 2111 is located at a rear side thereof when viewed from the side of the water tank 2100.

A distance from a center of the valve hole 2111 to a front surface of the water tank (in this embodiment, a tank front wall described below) is larger than a distance from the center of the valve hole 2111 to a rear surface of the water tank (in this embodiment, a first rear wall described below). The valve hole 2111 is defined at a rear side of the water tank 2100, thereby minimizing leakage from the water tank valve 2150 when the tilting assembly is operated.

While the tilting assembly is operating, the water tank valve 2150 is quickly closed when the water tank 2100 is quickly spaced apart from the water supply assembly 2200. As a front side of the water tank 2100 is tilted forward with respect to a lower side thereof, the water tank valve 2150 is preferably disposed at the rear side thereof.

The tank upper body 2130 is coupled to the upper surface of the tank middle body 2120. The tank upper body 2130 has a rectangular shape when viewed from the top.

The tank upper body 2130 is opened in the vertical direction. The tank upper body 2130 defines an upper body opening 2131 that communicates with the middle body upper opening 2121. The middle body opening 2121 is disposed under the upper body opening 2131.

The water tank handle 2140 is rotatably assembled to the tank upper body 2130.

The water tank handle 2140 is disposed inside the tank upper body 2130 and is hidden from the user when the water tank handle 2140 is stored in the lower cabinet 120.

The water tank valve 2150 functions as a check valve and is structurally optimized for the structure in this embodiment.

When the water tank valve 2150 disposed in the water tank 2100 is supported on the water supply assembly 2200, a lower side of the valve core 2152 contacts a valve supporter 2250 described below.

When the valve core 2152 is supported in contact with the valve supporter 2250, the water tank valve 2150 including the diaphragm 2154 is disposed on the valve supporter 2250 and the remaining components of the water tank 2100 except for the water tank valve 2150 are moved downward.

When the water tank valve 2150 is supported on the valve supporter 2250, the diaphragm 2154 opens the valve hole 2111. Meanwhile, when the water tank 2100 is separated from the water supply assembly 2200, the diaphragm 2154 closes the valve hole 2111 by pressure of water.

### <<Configuration of water supply assembly>>

The water supply assembly 2200 supplies water from a water tank 2100 to a steam generator 2300. The water supply assembly 2200 supplies the water to the steam generator 2300 by opening a water tank valve 2150 of the water tank 2100 only when the water tank 2100 is supported on the water supply assembly 2200.

The water supply assembly 2200 supports the water tank 2100 and provides a flow path through which water flows from the water tank 2100 to the steam generator 2300. In addition, the water supply assembly 2200 may open and close the water tank valve 2150 based on a level of water stored in the steam generator 2300. In this embodiment, the water tank valve 2150 is opened and closed by a mechanical arrangement, not based on an electrical signal. When the water tank valve 2150 is opened and closed electrically, an electric wire may be exposed to moisture or water, which may cause malfunction and safety issues.

In this embodiment, the water tank valve 2150 is opened and closed using the mechanical coupling relation, thereby minimizing use of electricity for a water contact portion and preventing the malfunction and safety accidents.

In addition, the water supply assembly 2200 functions to provide a tilting angle of the water tank 2100 when the water tank 2100 is tilted by the tilting assembly. In addition, the water supply assembly 2200 suppresses excessive tilting of the water tank 2100.

The water supply assembly 2200 includes a supply chamber housing 2210 disposed in the cabinet assembly 100 (in this embodiment, on a base) to temporarily store the water supplied from the water tank 2100 in a supply chamber 2211 and supply the water stored in the supply chamber 2211 to the steam generator 2300, a supply floater 2220 accommodated in the supply chamber 2211 of the supply chamber housing 2210 and vertically moving based on a level of water stored in the supply chamber 2211, a supply support body 2230 disposed on the supply chamber housing 2210, covering an upper surface of the supply chamber 2211, defining a portion of a supply flow path 2231 to guide water supplied from the water tank 2100 to the supply chamber 2211, and to support the water tank 2100 when the water tank 2100 is tilted and provide a tilting angle, a valve supporter 2250 disposed on the supply support body 2230, to open the water tank valve 2150 in contact with the water tank valve 2150 of the water tank 2100 when the water tank 2100 is supported and provide a portion of the supply flow path 2231 to guide, to the supply chamber 2111, the water discharged from the water tank valve 2150, a supply tilting cover 2260 to detachably support the water tank 2100, disposed between the water tank 2100 and the supply support body 2230, rotatable relative to the supply support body 2230 when the water tank is tilted, that penetrates the water supply valve of the water tank, and to supply the water in the water tank to the supply chamber 2111, and a water bellows 2240 disposed between the supply tilting cover 2260 and the supply support body 2230, connecting the supply tilting cover 2260 to the supply support body 2230, accommodating the valve supporter 2250, and to guide water supplied from the supply tilting cover 2260 to the supply chamber 2211 via a supply flow path 2231 of the supply support body 2230.

The water tank valve 2150 is disposed at a lower portion of the water tank 2100, a valve supporter 2250 and a supply support body 2230 are each disposed under the water tank valve 2150, the supply floater 2220 is disposed below the valve supporter 2250, and the supply floater 2220 moves vertically within a height of the supply chamber 2211.

The water in the water tank 2100 flows to the supply chamber 2211 through the water tank valve 2150, the water bellows 2240, and the supply flow path 2231. The supply chamber 2211 temporarily stores the supplied water and the water flows to the steam generator 2300 by potential energy due to its own weight.

### <Configuration of supply chamber housing>

The supply chamber housing 2210 is disposed on an upper surface of a base 130 of a cabinet assembly 100. The supply chamber housing 2210 temporarily stores water supplied from a water tank and provides the stored water to a steam generator 2300. The supply chamber housing 2210 provides an installation space of the supply floater 2220. The supply floater 2220 may move vertically in the supply chamber housing 2210.

The tilting assembly utilizes an installation structure of the supply chamber housing 2210 to tilt the water tank 2100. The supply chamber housing 2210 is a component to which a tilting assembly described below is assembled, and the structure thereof is described in more detail when explaining the tilting assembly.

When the humidification assembly 2000 is not used (e.g., in summer when humidity is high or when water is stored in a water tank for a long time period), all the water in the humidification assembly 2000 including the water tank 2100 does not remain inside, but is drained to the outside.

For the drainage, this embodiment provides a structure in which the water supplied from the water tank 2100 does not remain during the flow, but may flow by its own weight.

The valve supporter 2250 is disposed under the water tank valve 2150. The valve supporter 2250 interferes with the water tank valve 2150 when the water tank 2100 is supported on a water supply assembly 2200 and opens the water tank valve 2150.

The valve supporter 2250 has a pointed upper side and supports a valve core 2152 of the water tank valve 2150.

When the water tank 2100 is supported on the water supply assembly 2200, the valve supporter 2250 interferes with the valve core 2152 to push the water tank valve 2150 upward and open a valve hole 2111.

When the valve hole 2111 is opened, the water in the water tank 2100 flows to a supply support body 2230.

In this embodiment, as the water tank 2100 is tilted forward, the second supporter 2236b provides a tilting inclined surface 2237 with a high rear side and a low front side. The tilting inclined surface 2237 is provided on an upper surface of the second supporter 2236b. The tilting inclined surface 2237 is inclined from the rear side thereof toward the front lower side thereof.

The tilting inclined surface 2237 forms a predetermined tilting angle with a lower surface of the water tank 2100. The tilting inclined surface 2237 may have a tilting angle that is greater than or equal to 10 degrees and less than or equal to 45 degrees. When the water tank 2100 is supported on the tilting inclined surface 2237, the water tank 2100 may not be conducted. In addition, when the water tank 2100 is supported on the tilting inclined surface 2237, a water tank handle 2140 is exposed to a user, rotates upward, and is unfolded.

The water bellows 2240 is made of elastic material. The water bellows 2240 is coupled to each of the supply tilting cover 2260 and the supply support body 2230, and provides water discharged from the water tank to the supply support body 2230.

The water bellows 2240 prevents leakage of water discharged from the water tank 2100. When the water tank 2100 is tilted, the water bellows 2240 is elastically deformed and stretched. The water bellows 2240 connects the supply tilting cover 2260 to the supply support body 2230 even when the water tank is tilted.

In this embodiment, the water bellows 2240 has a corrugated pipe shape.

The supply floater 2220 is housed in the supply chamber 2211 and moves in a vertical direction based on a level of water in the supply chamber 2211.

When the level of water in the supply chamber 2211 is increased to a reference value or more, the supply floater 2220 closes a valve hole 2258. When the valve hole 2258 is closed, the water is not supplied to the supply chamber 2211, and the water in the supply chamber 2211 flows to a steam generator 2300 through a chamber housing pipe 2214.

As the water flows from the supply chamber 2211 to the steam generator 2300, the level of water in the supply chamber 2211 is lowered and a height of the supply floater 2220 is lowered. Therefore, the valve hole 2258 may be opened.

In this embodiment, water flowing from the water tank 2100 to the supply chamber 2111 undergoes two control processes.

First, the water tank valve 2150 opens and closes the valve hole 2111 to control water flow. Subsequently, a floater valve 2270 opens and closes a middle hole 2258 to control water flow.

As the water discharged from the water tank 2100 flows into the supply chamber 2111 through the two opening and closing processes, it is possible to prevent oversupply of water. Specifically, the supply floater 2220 additionally controls the water supply, thereby blocking the oversupply of the water to the steam generator 2300.

In addition, the supply chamber 2211 may accommodate a water level sensor to detect a water level.

### <<Configuration of steam generator>>

The steam generator 2300 receives water from the water supply assembly 2200 and generates steam. As the steam generator 2300 generates the steam by heating the water, the steam generator 2300 may provide sterilized steam.

The steam generator 2300 includes a steam housing 2310, a steam heater 2320 accommodated in the steam housing 2310 and to generate heat by applied power, a water supplier 2314 accommodated in the steam housing 2310, connected to a chamber housing pipe 2214 of the water supply assembly 2200, and to receive water, a steam discharger 2310 accommodated in the steam housing 2310, connected to the steam guide 2400, and to supply steam generated inside to the steam guide 2400, and an air suction portion 2318 accommodated in the steam housing 2310, connected to the humidification fan 2500, and to receive filtered air inside the cabinet assembly 100 from the humidification fan 2500.

The steam housing 2310 is closed from the outside. Only the water supplier 2314 and the steam discharger 2316 communicate with the inside of the steam housing 2310. The steam housing 2310 is disposed on a base 130.

In this embodiment, the water supplier 2314 is accommodated in lower steam housings 2310, 2312 and the steam discharger 2316 is accommodated in upper steam housings 2310, 2311.

The water supplier 2314 protrudes from the upper steam housings 2310, 2311 toward the water supply assembly 2300. The water supplier 2314 is connected to the chamber housing pipe 2214 and is arranged in a transverse direction. In the present embodiment, the water supplier 2314 has a hollow pipe shape.

Water in the supply chamber 2211 is introduced into the water supplier 2314 by its own weight. For the water introduction, the water supplier 2314 is disposed lower than the chamber housing pipe 2214. In particular, the water supplier 2314 is disposed at a same height as or at a height lower than an outer side 2214b of the chamber housing pipe 2214.

In particular, the water supplier 2314 is connected to a lowermost side of the lower steam housings 2310, 2312. In this embodiment, an additional valve is not disposed in the water supplier 2314.

As the water supplier 2314 and the chamber housing pipe 2214 communicate with each other, a level of water in the supply chamber 2211 may be the same as the steam housing 2310.

Specifically, when a sufficient amount of water is supplied to the inside of the steam housing 2310, the water level in the supply chamber 2211 may be the same as the steam housing 2310, a level of water in a supply floater 2220 of the water supply assembly 2200 is increased based on a rise in water level of supply chamber 2211, and the supply floater 2220 may close a middle hole 2258 through which water is supplied.

In this embodiment, the chamber housing pipe 2214 is disposed lower than a height of the steam heater 2320. The chamber housing pipe 2214 is disposed lower than a maximum water level of the steam generator 2300.

The middle hole 2258 is provided higher than the maximum water level of the steam generator 2300. In this embodiment, the middle hole 2258 is spaced apart from an upper side of the steam heater 2320 by a separation distance (H).

The steam discharger 2316 communicates with the inside of the upper steam housings 2310, 2311. The steam discharger 2316 penetrates the upper steam housing 2311 in the vertical direction. The steam discharger 2316 protrudes upward from an upper surface of the upper steam housings 2310, 2311 for connection to the steam guide 2400.

The air suction portion 2318 is accomodated in the steam housing 2310, more specifically, is accommodated in the upper steam housings 2310, 2311. The air suction portion 2318 communicates with the inside of the upper steam housings 2310, 2311 and the air supplied from the humidification fan 2500 is introduced to the air suction portion 2318.

The air suction portion 2318 protrudes upward from the upper surface of the upper steam housings 2310, 2311 for connection to the humidification fan 2500.

In this embodiment, the air suction portion 2318 is disposed behind the steam discharger 2316. The air suction portion 2318 is disposed closer to the humidification fan 2500 than the steam discharger 2316.

The air suction portion 2318 is connected to the humidification fan 2500 and receives filtered air from the humidification fan 2500. The air suction portion 2318 receives air filtered through the filter assembly 600. The filtered air supplied to the air suction portion 2318 is introduced into the steam housing 2310 and is discharged to the steam discharger 2316 together with the steam inside the steam housing 2310.

When general air, not filtered air, is introduced into the steam housing 2310, mold and the like are highly likely to be reproduced inside the steam housing 2310.

In this embodiment, as the air supplied to the inside of the steam housing 2310 is limited to the filtered air, it is possible to minimize contamination of the inside thereof due to virus or mold when the steam generator 2300 is not operated.

According to the present embodiment, as airflow of the humidification fan 2500 is supplied to an inside of the steam generator 2300 and pushes the steam out of the steam housing 2310, the steam generator 2300 may maximize a flow pressure of steam.

In another embodiment, when the humidification fan has a structure to suction steam at an outside of the steam housing 2310, the steam inside the steam housing 2310 may not be smoothly discharged.

When the steam generated by the steam generator 2300 does not flow quickly to side discharge outlets 301 and 302, dew condensation may occur during the steam flow.

In the present embodiment, the humidification fan 2500 supplies air at the air suction portion of the steam generator 2300, thereby minimizing the dew formation generated during the steam flow. In addition, in the present embodiment, the air in the humidification fan 2500 pushes the steam inside the steam housing 2310 out of the steam housing 2310, thereby obtaining a sufficient airflow rate.

In particular, in this embodiment, even if dew condensation occurs during the steam flow, the flow rate of air is sufficiently obtained to flow the steam, thereby naturally evaporating the condensed water with the airflow rate.

The steam guide 2400 supplies the steam of the steam generator 2300 to a discharge flow path. The discharge flow path includes an air flow path along which air flows by a long-distance fan assembly 400 and an air flow path along which air flows by a short-distance fan assembly 300.

In the present embodiment, the discharge flow path is defined in the cabinet assembly 100 and is referred to as a flow path along which air that has passed through the filter assembly 600 flows before the air is discharged to an outside of the cabinet assembly 100.

In this embodiment, the steam guide 2400 guides the steam generated by the steam generator 2300 to the side discharge outlets 301 and 302. The steam guide 2400 provides an additional flow path separated from the air inside the cabinet assembly 100. The steam guide 2400 may have a tube shape or a duct shape.

In the present embodiment, the diffuser is disposed at the side discharge outlet or may be disposed at the front discharge outlet. That is, the installation position of the diffuser is not limited to the side discharge outlet.

The side discharge outlets 301 and 302 discharge air in a forward and rightward direction and in a forward and leftward direction and humidified air is discharged to a space in front of the side discharge outlets 301 and 302. When the humidified air is discharged to a space in front of the side discharge outlets 301 and 302, the humidified air may flow farther.

When the humidification assembly 2000 according to the present embodiment provides humidification, an arrival distance of moisture does not depend only on an output of the humidification fan 2500. When relying only on the output of the humidification fan 2500, capacity of the humidification fan 2500 may be increased or the humidification fan 2500 may be operated at a high speed to flow moisture further.

In this embodiment, when the humidification assembly 2000 is operated, moisture may flow further together with a flow of air by the short-distance fan assembly 300 and the moisture and the air may flow further. In this case, humidification may be provided to a remote place in the indoor space even when the humidification fan 2500 having a small output capacity is used.

The humidified air may flow further when the diffuser outlet 2431 is defined at a front side of each of the side discharge outlets 301 and 302, not at a rear side of each of the side discharge outlets 301 and 302.

### <<Configuration of humidification fan>>

The humidification fan 2500 suctions filtered air that has passed through a filter assembly 600, supplies the filtered air to a steam generator 2300, and flows the filtered air together with the steam generated by the steam generator 2300 to a steam guide 2400.

The humidification fan 2500 forms airflow to discharge steam and filtered air (referred to as humidified air in this embodiment) from diffusers 2430 and 2440.

The humidification fan 2500 includes a humidification fan housing 2530 to suction air filtered through the filter assembly 600 and guide the suctioned filtered air to the steam generator 2300, a clean suction duct 2540 having a lower side connected to the humidification fan housing 2530, an upper side disposed at a front side of the filter assembly 600 to provide the air filtered through the filter assembly 600 to the humidification fan housing 2530, a humidification impeller 2510 accommodated in the humidification fan housing 2530 and to flow the filtered air of the humidification fan housing 2530 to the steam generator 2300, and a humidification motor 2520 accommodated in the humidification fan housing 2530 and to rotate the humidification impeller 2510.

The clean suction duct 2540 provides the filtered air that has passed through the filter assembly 600 to the humidification fan housing 2530.

The filter assembly 600 is disposed in the upper cabinet 110 and the humidification fan 2500 is disposed in the lower cabinet 120, so there is a height difference between filter assembly 600 and the humidification fan 2500. That is, the filter assembly 600 is disposed above the humidification fan 2500.

In particular, the filtered air that has passed through the filter assembly 600 flows to the short-distance fan assembly 300, but does not flow to the lower cabinet 120 or is difficult to flow to the lower cabinet 120. Specifically, as the lower cabinet 120 does not define an air discharging portion, the filtered air does not flow into the lower cabinet 120 or circulate through the inside of the lower cabinet 120 unless air is artificially supplied.

In addition, as a drain fan 140 is disposed under the upper cabinet 110 to support a heat exchange assembly and collect condensed water, there are a lot of restrictions on flow of the filtered air in the upper cabinet 110 to the lower cabinet 120.

An upper side of the clean suction duct 2540 is disposed inside the upper cabinet 110 and a lower side thereof is disposed inside the lower cabinet 120. That is, the clean suction duct 2540 provides a flow path to flow the filtered air inside the upper cabinet 110 into the lower cabinet 120.

The humidification fan housing 2530 includes a humidification fan housing 2550 coupled to the clean suction duct 2540, to suction the filtered air, and defining a first suction space 2551, a second humidification fan housing 2560 coupled to the first humidification fan housing 2550, to receive filtered air from the first humidification fan housing 2550, defining a second suction space 2561, accommodating the humidification impeller 2510, and to guide the filtered air to the steam generator 2300 based on an operation of the humidification impeller 2510, a first suction open surface 2552 provided in the first humidification fan housing 2550, that communicates with the first suction space 2551, and having an opening that is opened to one side (in this embodiment, an upper side), a second suction open surface 2562 provided in the second humidification fan housing 2560, that communicates with the second suction space 2561, and having an opening that is opened to the other side (in this embodiment, a lower side), a first suction space discharger 2553 that passes through the first humidification fan housing 2550 and the second humidification fan housing 2560 and communicates the first suction space 2551 with the second suction space 2561, and a motor installation portion 2565 accommodated in the humidification fan housing 2560 and to install the humidification motor 2520.

The first humidification fan housing 2550 includes the first suction open surface 2552 that is opened upward. The clean suction duct 2540 is connected to the suction open surface 2552. Meanwhile, the second humidification fan housing 2560 includes a second suction open surface 2562 that is opened downward.

In this embodiment, the opening direction of the first suction open surface 2552 is opposite to the opening direction of the second suction open surface 2562.

A lower surface 2554 of the first humidification fan housing 2550 has a round shape and is disposed below the first suction space discharger 2553. The upper surface 2564 of the second humidification fan housing 2560 has a round shape and is disposed above the first suction space discharger 2553.

A motor shaft (not shown) of the humidification motor 2520 passes through the second humidification fan housing 2560 and is assembled to the humidification impeller 2510.

The motor installation portion 2565 protrudes rearward from the second humidification fan housing 2560 and the humidification motor 2520 is inserted into and disposed in the motor installation portion 2565.

The first humidification fan housing 2550 including the first suction space 2551 and the second humidification fan housing 2560 including the second suction space 2561 may be respectively manufactured and then assembled to each other.

In this embodiment, the humidification fan housing 2530 is manufactured by assembling three components to simplify the assembly structure and reduce the manufacturing cost thereof.

The humidification fan housing 2530 includes a first humidification fan housing 2531 that surrounds a front side of the first suction space 2551 and constitutes a portion of the first humidification fan housing 2550, a second humidification fan housing 2532 that surrounds a rear side of the first suction space 2551 and a front side of the second suction space 2561, accommodating the first suction space discharger 2553, and constituting the remaining of the first humidification fan housing 2550 and a portion of the second humidification fan housing 2560, and a third housing 2533 that surrounds a rear side of the second suction space 2561, accommodating the motor installation portion 2565, and constituting the remaining portion of the second humidification fan housing 2560.

The second humidification fan housing 2532 is used both in the first humidification fan housing 2550 and the second humidification fan housing 2560, thereby simplifying the number of components and reducing manufacturing costs.

The second humidification fan housing 2532 accommodates the first suction space discharger 2553. The first suction space discharger 2553 passes through the second humidification fan housing 2532 in a forward and rearward direction.

The first suction space discharger 2553 protrudes toward the humidification impeller 2510 and has a circular shape.

The second humidification fan housing 2532 accommodates the first suction space discharger 2553 and includes an orifice 2534 that protrudes toward the humidification impeller 2510.

A first suction space 2551 is defined at a front side of the second humidification fan housing 2532 and a second suction space 2561 is defined at a rear side thereof.

The humidification impeller 2510 is a centrifugal fan to suction air at a central portion thereof and discharge air in a circumferential direction. The air discharged from the humidification impeller 2510 flows to the steam generator 2300 through the second humidification fan housing 2560.

A flow of the filtered air based on the driving of the humidification motor 2520 is described as follows.

When the humidification motor 2520 is driven, the humidification impeller 2510 coupled to the humidification motor 2520 is rotated. When the humidification impeller 2510 is rotated, air flow is generated in the humidification fan housing 2530, and filtered air is suctioned through the clean suction duct 2540.

The filtered air suctioned through the clean suction duct 2540 flows to the second humidification fan housing 2560 through the first suction space 2551 and the first suction space discharger 2253 of the first humidification fan housing 2550. The air moved into the second humidification fan housing 2560 is pressurized by the humidification impeller 2510, flows downward along the second humidification fan housing 2560, and then flows into the steam generator 2300 through the second suction open surface 2562.

The filtered air flowing into the steam housing 2310 through the air suction portion 2318 of the steam generator 2300 is discharged to the steam discharger 2316 together with the steam generated by the steam generator 2300.

The humidified air discharged from the steam discharger 2316 is branched into a first branch guide 2410 and a second branch guide 2420 from a main steam guide 2450.

The humidified air flowing to the first branch guide 2410 is discharged to the first side discharge outlet 301 through the first diffuser 2440 and the humidified air flowing to the second branch guide 2420 is discharged to the second side discharge outlet 302 through a second diffuser 2450.

The humidified air discharged from the first side discharge outlet 301 is diffused to the left side of the cabinet assembly 100 together with an airflow generated by the short-distance fan assembly 300 and the humidified air discharged through the second side discharge outlet 302 is diffused to the right side of the cabinet assembly 100 together with an airflow generated by the short-distance fan assembly 300.

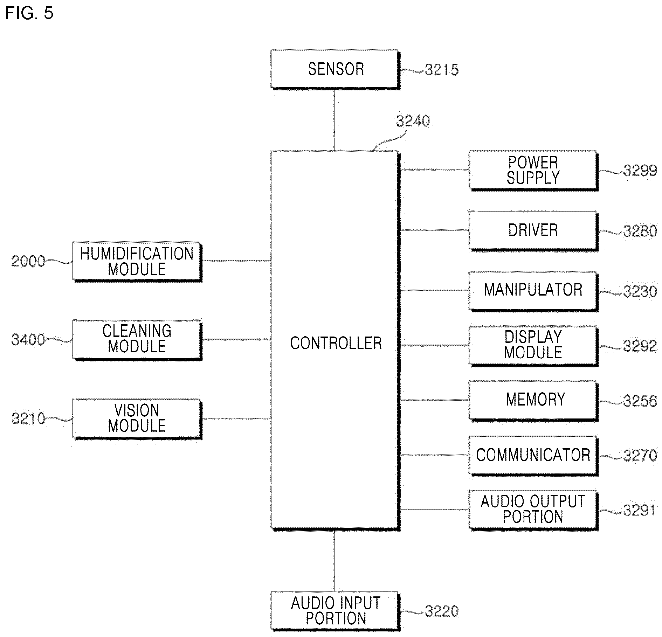

FIG. 5 is a schematic block diagram of components of an air conditioner according to an embodiment of the present disclosure.

As shown in FIG. 5, the air conditioner includes a sensor 3215, a power supply 3299, a driver 3280, a manipulator 3230, a display module 3292, a memory 3256, and a communicator 3270, an audio output portion 3291, an audio input portion 3220, a vision module 3210, a cleaning module 4400, a humidification module 2000, and a controller 3240 to control overall operations.

The power supply 3299 supplies operating power to a main body. The power supply 3299 rectifies and smoothes the connected commercial power source to generate and supply a voltage required by each component. The power supply 3299 prevents inrush current and generates a constant voltage. In addition, the power supply 3299 may supply the operating power to an outdoor unit (not shown).

The driver 3280 provides a driving force to rotate a long-distance fan assembly 400. In addition, the driver 3280 provides power to a moving means (not shown) to move the long-distance fan assembly 400. In addition, the driver 3280 controls opening and closing of a valve disposed therein. In some cases, the driver 3280 may provide a driving force to slidably move a front panel 11 in a leftward direction or a rightward direction. The driver 3280 may include a long-distance fan assembly driver, a moving means driver, a valve driver, and a front panel driver.

The manipulator 3230 includes at least one of a button, a switch, or a touch input means to input a user command or predetermined data to the indoor unit.

The display module 3292 has display means such as LCD, LED, and OLED and may include a touch screen layered with a touch pad. The display module 3292 indicates operation set or operation information of the indoor unit with a combination of at least one of letters, an image, a special character, a symbol, an emoticon, or an icon. In addition, the display module 3292 may further include a lighting to indicate an operation state according to lighting or non-lighting, a lighting color, and flickering or non-flickering.

The audio output portion 3291 outputs voice guidance, a predetermined warning sound, and an effect sound. The audio output portion 3291 includes a buzzer or a speaker. The audio input portion 3220 receives and recognizes voice of a user and inputs a voice command to the controller 3240. The audio input portion 3220 includes at least one microphone.

The memory 3256 stores control data to control an operation of the indoor unit, operation mode data, data sensed by the sensor 3215, data transmitted and received through the communicator, data input by the manipulator, output data, and data to determine abnormality of operation. The memory 3256 stores data that may be read by a micro processor and may include a hard disk drive (HDD), solid state disk (SSD), silicon disk drive (SDD), ROM, RAM, CD-ROM, magnetic tapes, floppy disks, optical data storage devices, and the like.

The communicator 3270 includes at least one communication module and transmits and receives data by a wired or wireless communication.

The communicator 3270 transmits and receives data to and from an outdoor unit (not shown), and receives data from a remote controller (not shown). In addition, the communicator 3270 may be connected to a predetermined network to communicate with an external server or a terminal. The communicator 3270 performs short-range wireless communication such as Zigbee, Bluetooth, and infrared rays, and includes a communication module such as Wi-Fi and WiBro to transmit and receive data.

The sensor 3215 includes a plurality of sensors to input measured data to the controller 3240. The sensor 3215 includes a proximity sensor 17, a temperature sensor, a pressure sensor, and a humidity sensor, and includes a water tank detector.

The proximity sensor 17 detects a person or an object approaching within a predetermined distance. The proximity sensor 17 may be disposed at a lower portion of the main body and a front portion of a base, and may also be disposed adjacent to the display module 3292. The proximity sensor inputs an approach signal to the controller 3240 if a predetermined object or person approaches within the predetermined distance.

The temperature sensor is disposed at a suction inlet to measure an indoor temperature, is disposed in the main body to measure a heat exchange temperature, is disposed in any one of the discharge outlets to measure a temperature of discharged air, and is disposed in a refrigerant pipe to measure a refrigerant temperature. The humidity sensor measures humidity of indoor air.

The vision module 3210 includes at least one image acquisition portion and captures an indoor environment and detects a location of a user. In addition, the vision module may detect indoor intrusion according to an operation mode. The vision module 3210 is disposed on a front panel 11, and in some cases, may be disposed on an upper panel of the cabinet.

The cleaning module 4400 is disposed in the filter and cleans foreign substances in the filter. The cleaning module includes a cleaning robot (not shown). The cleaning robot suctions the foreign substances from the filter while moving along a surface of the filter. In addition, the cleaning robot may sterilize the filter using a sterilization lamp while cleaning the filter. The cleaning module 4400 may further include a position sensor to detect the position of the robot cleaner.

The humidification module 2000 receives water from the water tank 2100, performs humidification to provide moisture, and discharges humidified air to the outside. The humidification module 2000 humidifies the air by generating steam and discharges the humidified air to the indoor space through the discharge outlet together with the conditioned air.

The humidification module 2000 may use a vibration method with vibration, a heating method, and a spray method to spray water, and various other humidification methods may be used. The humidification module may include a humidification assembly. It is specified that the same reference numeral is used for the humidification module and the humidification assembly.

The controller 3240 processes input and output data, stores data in a memory, and controls the communicator to transmit and receive the data. The controller 3240 sets the air conditioner to be operated based on set input to the manipulator, transmits and receives data to and from the outdoor unit, and controls the driver 3280 to discharge, to the indoor space, cold and hot air conditioned by the refrigerant supplied from the outdoor unit.

The controller 3240 controls the humidification module 2000 to discharge humidified air, controls the vision module 3210 to detect an occupant, and controls the cleaning module 4400 to clean a filter based on the set operation mode or the data measured by the sensor 3215.

In addition, the controller 3240 controls the heat-exchanged air to be discharged together with the humidified air according to the operation mode.

When setting a smart operation, the controller 3240 automatically switches a mode between a comfortable mode and a high speed mode, controls the heat-exchanged air and the humidified air to be discharged, and discharges the air with adjusted temperature and humidity to the indoor space.

The controller 3240 controls the operation in the high speed mode to reach to the set temperature in a short time period based on the detected temperature and humidity and controls the operation in the comfortable mode to maintain constant temperature and humidity.

The controller 3240 monitors the operation state of each module and controls the display module 3292 to indicate the operation state based on input data.

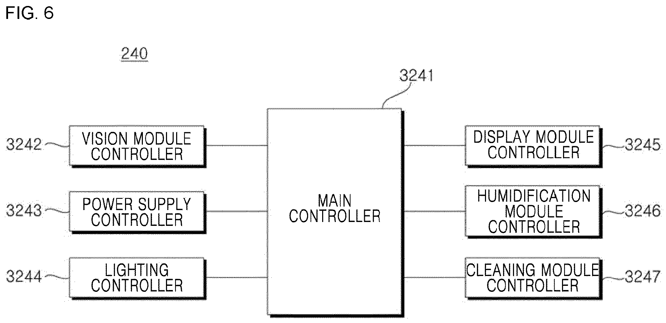

FIG. 6 is a block diagram of components of a processor for controlling an air conditioner according to an embodiment of the present disclosure.

As shown in FIG. 6, a controller 3240 may include one or a plurality of microprocessors.

The controller 3240 includes a main controller 3241, a vision module controller 3242, a power supply controller 3243, a lighting controller 3244, a display module controller 3245, a humidification module controller 3246, and a cleaning module controller 3247 according to functions thereof.

Each controller may include a microprocessor and may be disposed in a module. For example, a vision module 3210, a cleaning module 3400, and a humidification module may be controlled using a microprocessor. In addition, a microprocessor is disposed in each module, the vision module 3210 includes the vision module controller 3242 and the humidification module includes the humidification module controller to control operations thereof.

The main controller applies a control command to each controller, receives data from each controller and processes the data. The main controller and each controller may be connected to each other in a BUS format to transmit and receive the data.

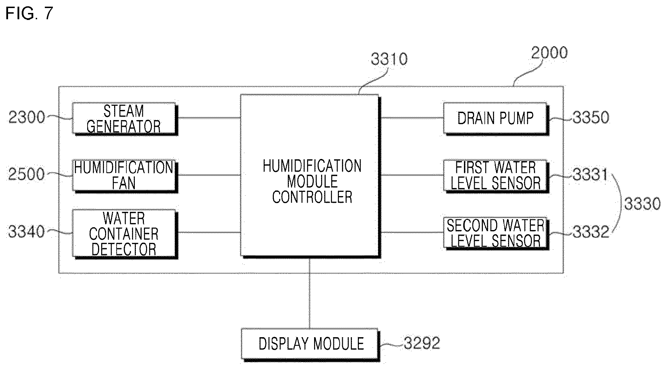

FIG. 7 is a schematic block diagram of components of a humidification module of an air conditioner according to an embodiment of the present disclosure.

As shown in FIG. 7, a humidification module 2000 includes a humidification assembly 2000.

The humidification module 2000 includes a steam generator 2300, a water level sensor 3330, a water tank detector 3340, a drain pump 3350, a temperature sensor, a humidification fan 2500, and humidification module controllers 3310 and 3246 to control an overall operation of the humidification module.

The steam generator 2300 includes the steam generator 2300 to generate humidified air by changing water supplied from a water tank 2100 to fine particles.

The steam generator 2300 includes a temperature sensor to prevent overheating, and a second water level sensor 332 is disposed.

The steam generator 2300 may use at least one of a heating type steam generator to generate steam by heating water, an ultrasonic type steam generator to generate humidified air by changing the water to fine particles by vibration using an ultrasonic vibrator, a compound type steam generator which is a combination of the heating type steam generator and the ultrasonic type generator, a centrifugal spray type steam generator to provide humidified air by spraying water, or a filter vaporization type steam generator to evaporate water using a wet filter.

The steam housing 2310 receives water automatically supplied from the water tank 2100.

The water level sensor 3330 is disposed in each of the water tank 2100 and the steam housing 2310 and detects a height of introduced water. A first water level sensor 331 may be disposed in the water tank 2100 and a second water level sensor 332 may be accommodated in the steam housing 2310.

The water level sensor 3330 detects the water level based on an amount of water in the water tank 2100, may output a full water level signal based on the water in the water tank exceeding a certain amount, and may output a zero water level signal based on no water in the water tank.

The water level sensor 3330 may measure a water level value by at least one electrode arranged vertically. When there is water in the water tank, the water level sensor may measure the water level based on a change in resistance value of the water.

In addition, the water level sensor 3330 may detect the water level by an electrode to which a signal is input according to the water level using a plurality of electrodes having different lengths, may detect the water level based on a signal which is detected when a plurality of sensors are arranged in a line with respect to a vertical axis of a surface of the water tank or a steam housing 2310, detect the water level based on a signal such as a ultrasonic wave, and detect the water level based on changes in position of a magnet owing to the water level by inserting the magnet to a hose accommodated in the water tank 2100 or the steam housing 2310.

The water level sensor 3330 detects the water level by at least one method or by mixing a plurality of methods.

The water tank detector 3340 detects a mounted state of the water tank 2100.

The water tank detector 3340 detects that the water tank 2100 protrudes forward and an inlet is opened or that the water tank is separated from the main body. The humidification module controller 3310 may transmit a signal to the controller 3240 to output an error indicating that the water tank is not normally mounted on the display module 3292 based on the signal detected by the water tank detector 3340.

Based on the detection signal detected by the water tank detector 3340, the display module 3292 may display a water tank icon or a water tank image, a lamp may be turned on when the water tank is in an error state, or a guide message may be output to guide mounting of the water tank.

The drain pump 3350 is disposed in a drain hose connected to the steam housing 2310 of the steam generator 2300 to discharge water from the steam housing 2310, which is a water tank for steam, to the outside. The drain pump 3350 operates according to a control command of the humidification module controller 3310 and stops the operation thereof after the water is drained.

When the drain pump 3350 is operated, water is drained. In addition, when the drain pump 3350 is operated, the water in the steam housing 2310 is drained. At the same time, the drained water and the water supplied from the water tank to the steam housing 2310 by the water supply assembly 2200 are mixed together, flow into the drain pump, and are discharged through a drain hose by the drain pump.

As the water in the steam housing 2310 is mixed with the water in the water tank, a temperature may be decreased.

A flow path through which the water supply assembly 2200 supplies water to the steam housing 2310 is connected to a flow path through which water is drained from the steam housing to the drain pump to mix the heated water of the steam housing 2310 with the water in the water tank.

During drainage, the humidification controller may control the drain pump to drain the water after cooling the water based on the water temperature of the steam housing 2310 detected by the temperature sensor being higher than or equal to a set temperature. If the temperature of the water in the steam housing 2310 is equal to or higher than the set temperature, the drain hose or the drain pump may be damaged. Therefore, when the temperature is reduced to be equal to or less than the set temperature, the humidification controller controls the water to be drained.

The water supply assembly 2200 is installed in order to mix water in the steam housing 2310 and the water in a water container and introduces them into the drain pump. So, even if the temperature of water in the steam housing 2310 is less than the set temperature, water having a temperature lower than that of water in the steam housing 2310 may be introduced into the drain pump.

The humidification module controller 3310 controls to generate humidified air according to the control command of the controller 3240. When the humidification operation is set, the controller 3240 transmits the control command according to the humidification operation to the humidification module controller 3310, and the humidification module controller 3310 operates the steam generator 2300 based on the water level, the temperature, and the set mode to discharge humidified air.

The humidification module controller 3310 checks the state of the sensor during the humidification operation, and performs the humidification operation after the water boiling operation. In addition, the humidification module controller 3310 controls an overall humidification operation by performing steam sterilization after the humidification operation and performing water cooling after the humidification operation or the steam sterilization.

When a smart operation is set, the humidification module controller 3310 may operate the humidification module based on detected humidity.

The humidification module controller 3310 controls the water tank detector 3340 to detect whether the water tank is mounted.

The humidification module controller 3310 controls the water level sensor 3330 to determine an amount of water in the water tank. The humidification module controller 3310 outputs a no-water notification to supply water when there is no water in the water tank or the water level is equal to or less than a certain level.

In addition, when a water level display portion (not shown) is disposed at a portion of an inner panel 141 or the water tank 2100, the humidification module controller 3310 controls the water level detected by the water level sensor to be displayed on the water level display portion.

When the water tank is opened or is separated from, removed from the main body, and then is mounted on the main body, the humidification module controller 3310 determines that new water is supplied to the water tank, sets a water use time period, and counts a time period for which water remains in the water tank from a water supply time point.

In addition, when the water tank is mounted, the humidification module controller 3310 may compare the water levels before and after the water tank is mounted to determine whether new water is supplied.

The humidification module controller 3310 controls the drain pump 3350 to drain the water when the water remains in the water tank for a predetermined time period or more. The humidification module controller 3310 controls the water to be drained when the water in the water tank remains until the set use time. In addition, the humidification module controller 3310 may extend the use time period when the use time period is reached during the humidification operation.

The humidification module controller 3310 outputs a water drain notification on the display module, and after the water is drained, the humidification module controller 3310 outputs the no-water notification. In addition, the humidification module controller 3310 initializes the use time period after the water is drained.

When the water is normally supplied to the water tank, the humidification module controller 3310 controls a first valve to supply water to the steam housing 2310, and controls the steam generator 2300 to generate humidified air.

The humidification module controller 3310 may determine whether the water is supplied from the water tank to the steam housing 2310, identify whether the steam heater 2320 is normally operated based on a change in water temperature when the steam generator is operated, and output an error based on the identification result thereof.

The steam heater 2320 includes a plurality of heaters having different capacity, that is, heating value per hour. The steam heater 2320 may include a first heater having a first capacity and a second heater having a second capacity. The first heater may be set to have the larger capacity than that of the second heater.

In addition, when humidified air is generated, the humidification module controller 3310 discharges the humidified air to a discharge outlet through the steam guide 2400 connected to the steam discharger 2316 of the steam housing 2310. The steam guide 2400 is connected to the discharge outlet or an auxiliary vane and discharges the humidified air together with the heat-exchanged with to the indoor space. In addition, the steam guide 2400 may be connected to a flow path through which the heat-exchanged air flows and discharges together with the conditioned air through the discharge outlet.

The humidification module controller 3310 controls the steam generator to stop the operation thereof based on the water level of the water tank and the water level of the steam housing and outputs a no-water notification.

The humidification module controller 3310 generates a predetermined signal to output a water tank mount notification, a water drain notification, and a no water notification, transmits the signal to the display module, and outputs a notification with at least one of an icon, an image, a notification message, or lamp lighting.

In addition, the humidification module controller 3310 generates a predetermined signal and transmits the signal to the controller to output the water tank mount notification, the water drain notification, and the no-water notification, and the controller controls the display module 3292 to output a notification based on the signal.

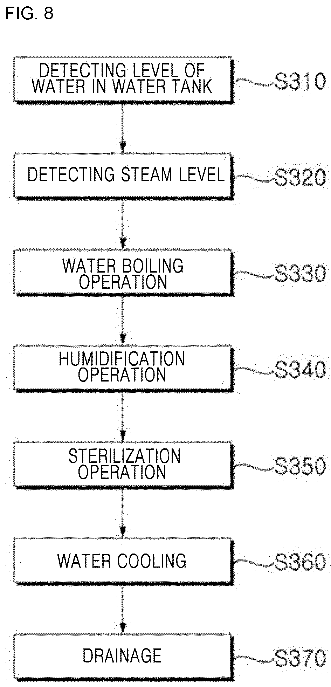

FIG. 8 is a flowchart of an operation for providing air humidified by an air conditioner according to an embodiment of the present disclosure.

As shown in FIG. 8, a humidification module 2000 sets a humidification operation, detects a water level of a water tank, and checks an amount of water for the humidification operation (S310). The humidification module may detect the water level of the water tank and determine a mounted state or a non-mounted state of the water tank.

In addition, the humidification module 2000 detects the water supplied to a steam housing, that is, a steam level (S320). The humidification module 2000 determines whether the water is supplied to the steam housing and the water level is a water level at which a heater may be operated.

If the water level is the level at which the heater may be operated, the humidification module 2000 may perform a water boiling operation (S330), which is a preparation operation, before performing the humidification operation.

If the water temperature reaches to a set temperature, for example, 100 Celsius degrees, the humidification module 2000 performs a humidification operation.

After the humidification operation is finished, the humidification module 2000 performs a steam sterilization operation of sterilizing a flow path through which steam flows by generating steam according to setting or non-setting of a sterilization mode (S350).

When the sterilization mode is not set or the steam sterilization operation is completed, the humidification module 2000 performs a water cooling operation to lower the temperature of the water in the steam housing (S360).

The humidification module 2000 generates the humidified air by driving the steam generator 2300 from the water boiling operation to the water cooling operation and controls a rotational speed of the humidification fan 2500. In addition, the humidification module 2000 may change the capacity or the number of heaters according to the operation and discharge the humidified air to the indoor space.

In addition, the humidification module 2000 may count a time period for which the water is accommodated in the water tank, and if the water remains for a predetermined time period or more, the humidification module 2000 may automatically drain the water (S370).

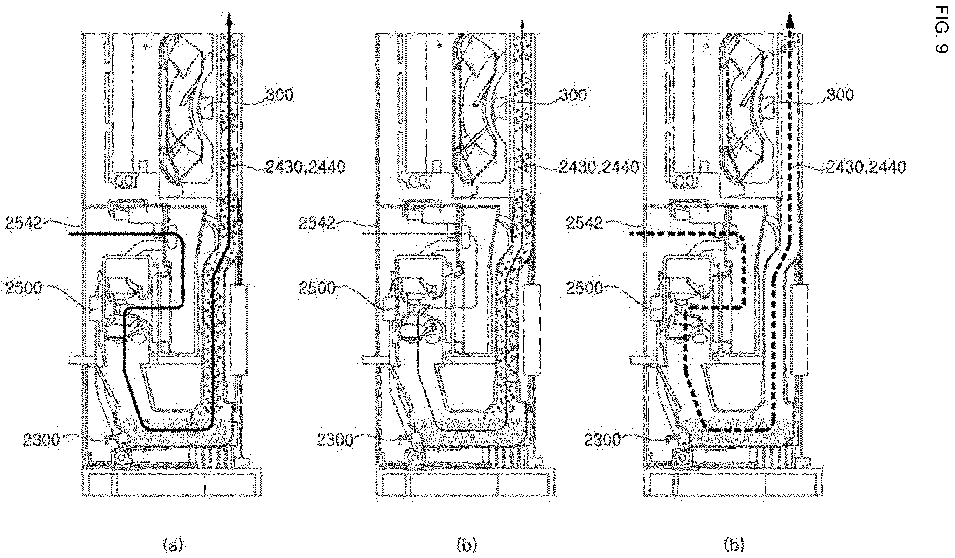

FIG. 9 show airflows generated according to operation steps of an air conditioner according to the embodiment of the present disclosure.

As shown in FIG. 9, humidified air flows according to the operation steps thereof.

(a) of FIG. 9 shows a flow of humidified air during a humidification operation. (b) of FIG. 9 shows a flow of humidified air during steam sterilization. (c) of FIG. 9 (c) shows an airflow during water cooling operation.

Filtered air is suctioned by an air suction portion 2542 and is introduced into a steam housing of a steam generator 2300.

The steam generator 2300 operates a heater during a water boiling operation, the humidification operation, and the steam sterilization operation to heat received water and generate humidified air. Meanwhile, during the water cooling operation, the steam generator turns off all heaters. However, during that water cooling operation, humidification may partially occur based on an increase in temperature of water due to the previous operation thereof.

The humidified air generated from the steam generator 2300 flows to a discharge outlet through a steam guide 2400.

A humidification fan 2500 and an indoor fan 300 are operated to discharge the humidified air generated from the steam generator through a discharge outlet. The humidification fan and the indoor fan are operated together to easily discharge the humidified air.

The humidification fan 2500 may be operated in three modes of sterilization wind, humidification wind, and drying wind during the humidification operation. A rotational speed of the humidification fan is set to increase in the sequence of the sterilization wind, the humidification wind, and the drying wind.

During the humidification operation, the humidification fan is operated with the humidification wind. When setting the humidification wind, the rotational speed of the humidification fan is determined based on the rotational speed of the indoor fan. In the case of steam sterilization, the humidification fan rotates with low-speed sterilization wind and slowly flows steam air. As the water cooling operation is an operation of reducing a temperature of received water and drying a steam guide and a discharge outlet, the humidification fan is operated with the drying wind, which is strong wind.

At a time of humidification operation of providing humidified air, a humidification module controller sets a speed of the humidification fan and the indoor fan, and the heater operation by the steam generator differently according to the operation modes thereof.

Accordingly, as shown in (a), (b), and (c) of FIG. 9, a wind direction and the flow of the humidified air may be set differently in the humidification operation, the steam sterilization operation, and the water cooling operation.

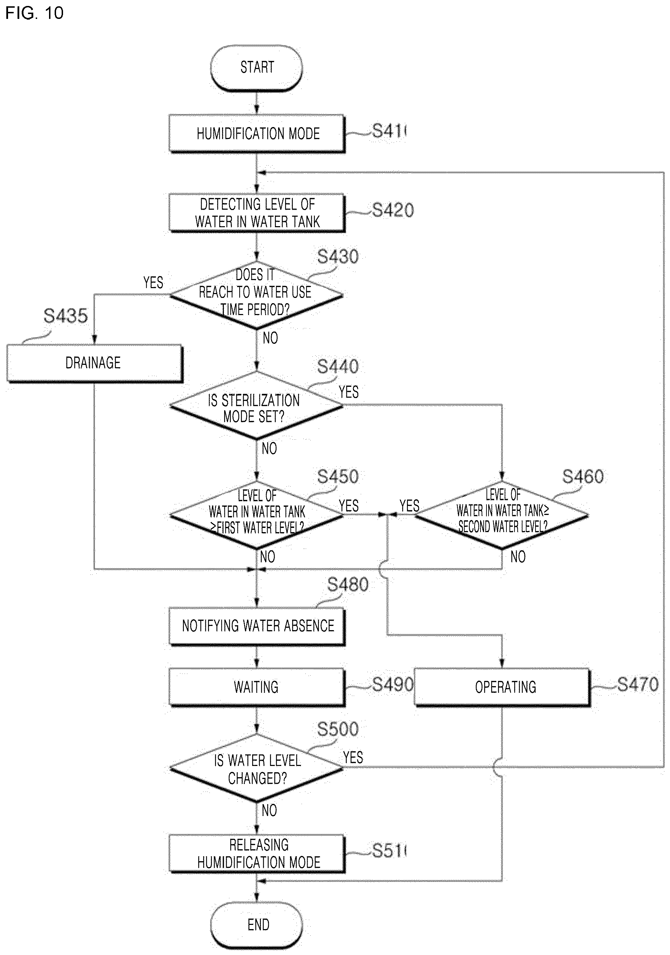

FIG. 10 is a flowchart of a method for managing water by an air conditioner according to an embodiment of the present disclosure.

As shown in FIG. 10, after a humidification mode is set, a humidification module 2000 detects a level of water in a water tank 2100 using a first water level sensor 3331 (S420).

In addition, a humidification module controller 3310 counts a use time period of the water accommodated in the water tank 2100 and determines whether the water use time period is reached to in order for water to not remain in a water container for a predetermined time period or more (S430).

Based on the water use time period being reached, the humidification module controller 3310 operates a drain pump to drain the water stored in the water tank and a steam generator (S435).

Meanwhile, the humidification module controller 3310 may extend the water use time period when the water use time period is reached during the humidification operation. When the humidification operation is finished according to setting, the humidification module controller 3310 may drain the water or may extend by a predetermined time period, and if the extended time period is reached, the humidification module controller 3310 may automatically drain the water.

After the water is drained, the humidification module controller 3310 transmits a no-water notification to the controller and controls a display module to display no-water warning.

Based on the water use time period being not reached, the humidification module controller 3310 determines whether a water level of the water tank is a water level at which the humidification operation may be performed according to setting or non-setting of a sterilization mode (S440).

In order to perform the sterilization mode, water with an amount larger than that of water in the humidification operation is needed. Therefore, the humidification module controller 3310 determines whether the water level of the water tank is equal to or greater than a first water level (S450).

Based on the sterilization mode being released, the humidification module controller 3310 determines whether the water level of the water tank is equal to or higher than a second water level which is set based on the amount of water for the humidification operation (S460).

The water level of the water tank may be determined by dividing into a plurality of levels and may be divided into a no water level, a minimum water level for humidification operation, a steam sterilization water level, and a full water level.

The first water level refers to a water level having a larger amount of water than that of the second water level. For example, the first water level is set to be higher than the second water level by about 250 to 500 cc. The second water level is set to a minimum water level at which the humidification operation may be performed.

When the sterilization mode is set, based on the water level of the water tank being less than the first water level, the humidification module controller 3310 determines a water state as a no water state. When the sterilization mode is released, based on the water level of the water tank being less than the second water level, the humidification module controller 3310 determines a water state as a no water state.

When the water is insufficient, the humidification module controller 3310 may transmit a no-water notification to the controller and control the display module to display no-water warning (S480).

After the no-water notification, the humidification module controller 3310 waits for a predetermined time period (S490) and determines whether new water is supplied to the water tank based on a change in water level detected by the first water level sensor (S500).

When the water is supplied, the humidification module controller 3310 performs a humidification operation based on the detected water level (S420 to S470).

Meanwhile, if the water is not supplied even after waiting for the predetermined time period, the humidification module controller 3310 releases the humidification mode (S510). In addition, it is possible to output a warning about the humidification mode release due to the water shortage.

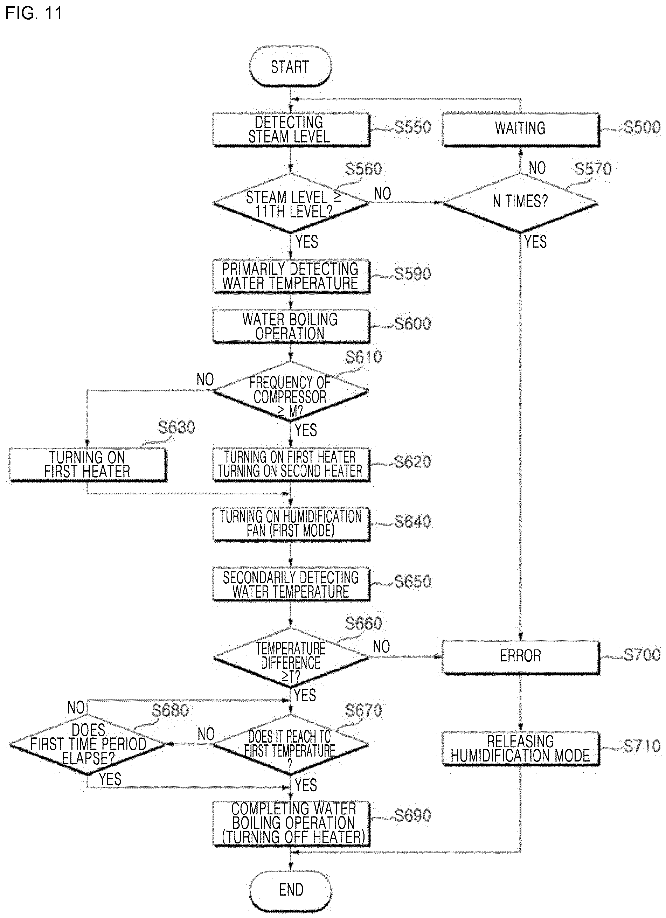

FIG. 11 is a flowchart of a method for performing a boiling water operation by an air conditioner according to an embodiment of the present disclosure.

As shown in FIG. 11, a humidification module 2000 performs a water boiling operation as a preparation operation before performing a humidification operation.

The humidification module controller 3310 detects a water level of the water tank and, if the humidification operation is possible, controls a second water level sensor to detect a level of water stored in steam housing, that is, a steam level (S550).

The humidification module controller 3310 determines whether the steam water level is equal to or higher than an 11th water level (S560). The 11th water level is set to be a level at which a heater of a steam generator is submerged in water to generate humidified air. If the heater is not submerged in water and is exposed, the heater may be overheated, which causes an accident. Therefore, the humidification module controller 3310 determines that the steam generator may be operated based on the steam level being equal to or higher than the 11th water level.

Based on the steam level being less than the 11th water level, the humidification module controller 3310 determines that the steam generator is in an inoperable state, and detects the level again (S550) after waiting for a predetermined time period (S580) for a predetermined number (S570).

The humidification module controller 3310 compares the water levels of the steam housing 2310 at predetermined time intervals and determines whether water is normally supplied based on a change in water level of the steam housing.

Water in a water tank 2100 is automatically supplied to a steam generator 2300 and is accommodated in the steam housing 2310. When the water level of the water tank 2100 is equal to or higher than the first water level, the steam housing generally has a full water level. However, when water in the water tank is newly supplied, supplying water from the water tank to the steam generator takes a certain time. Therefore, the humidification module controller 3310 may determine the water level change at the predetermined time intervals in consideration of the new water supply.

The humidification module controller 3310 may firstly determine whether the steam water level is equal to or higher than the 11th water level, wait for a first time period, and secondarily determine the water level. The first time period may be set as a time period from a time point of a no-water level of the steam housing to a time point of the 11th water level, which is reached by water supply from the water tank. After the first time period, the humidification module controller 3310 may determine the water level change a plurality of times in a unit of second time period that is shorter than the first time period. Based on the steam level reaching the 11th water level during the water level determination, the steam generator may immediately start a water boiling operation.

When the steam level does not reach to the 11th water level even after an elapse of the first time period or more in a state in which the water level of the water tank is equal to or higher than the first water level, the humidification module controller 3310 may determine that the water is not supplied from the water tank to the steam housing.

The humidification module controller 3310 may output an error about the water supply (S700). When an error occurs, the humidification module controller 3310 determines that the humidification module cannot perform the humidification operation and releases a humidification mode (S710).

When the water with the 11th water level or higher is stored in the steam housing, the humidification module controller 3310 firstly detects the water temperature (S590) and performs the water boiling operation (S600).

The humidification module controller 3310 operates a plurality of heaters of the steam generator based on frequency of a compressor. The humidification module controller 3310 operates both a first heater and a second heater (S620) based on operating frequency of the compressor being equal to or greater than a set frequency (M), and operates the first heater to generate humidified air based on the operating frequency of the compressor being less than the set frequency. If the operating frequency of the compressor is high, load is high, and accordingly, two heaters are both operated.

When the steam generator is operated, the humidification module controller 3310 controls the humidification fan to be operated (ON) at a first speed, which is a low speed (S640).

During the water boiling operation, the humidification fan may be operated at a first speed, that is, with low-speed sterilization wind. The humidification fan may be operated at a first speed of about 1000 to 120 rpm with the sterilization wind. In this case, air volume at a discharge outlet may be 0.25 CMM.

The water boiling operation is not for humidification, but is a preparation operation to quickly provide humidified air prior to the humidification operation which is a main operation. Therefore, the humidified air may flow slowly in the water boiling operation. In addition, in the water boiling operation, the humidified air may sterilize a steam guide and the discharge outlet prior to the humidification operation while slowly flowing. In addition, when the humidification fan is operated at a high speed during the water boiling operation, water in the steam housing may flow backward by the humidification fan. Therefore, it is preferable to rotate the humidification fan at a low speed.

After the heater is operated, in a set time period, a temperature sensor senses the water temperature again (S650). For example, the temperature sensor may detect a temperature difference after about 5 to 10 minutes. For example, the temperature sensor may detect the water temperature again after 7 minutes.

The humidification module controller 3310 compares temperature values before and after operating the heater and determines whether a water temperature difference is equal to or higher than a set temperature (T) (S660). Based on the temperature difference being less than the set temperature, the humidification module controller 3310 determines a state of the heater as an error state (S700). Based on the error occurrence, the humidification module controller 3310 determines that the humidification module cannot perform the humidification operation and releases a humidification mode (S710). The set temperature (T) may be preferably set to have a minimum value of the temperature difference which may be generated by heating water received in the steam generator by a heater for 5 to 10 minutes. For example, the set temperature may be about 10 Celsius degrees.

Meanwhile, based on the temperature difference being equal to or higher than the set temperature, the humidification module controller 3310 determines that the heater is operated normally and maintains the operation thereof to generate humidified air.

The humidification module controller 3310 detects the water temperature and determines whether the water temperature reaches to a first temperature, which is a target temperature (S670).

The humidification module controller 3310 stops the operation (OFF) of the heater and completes the water boiling operation (S690) based on the water temperature reaching to the first temperature, which is the target temperature.

The humidification module controller 3310 operates the heater until the water temperature reaches to the target temperature and counts a time period from a heater operation time point to determine whether an 11th time period is reached (S680). Based on the temperature not reaching to the target temperature even after an elapse of the 11th time period, the humidification module controller 3310 stops the operation of the heater and completes the water boiling operation (S690).

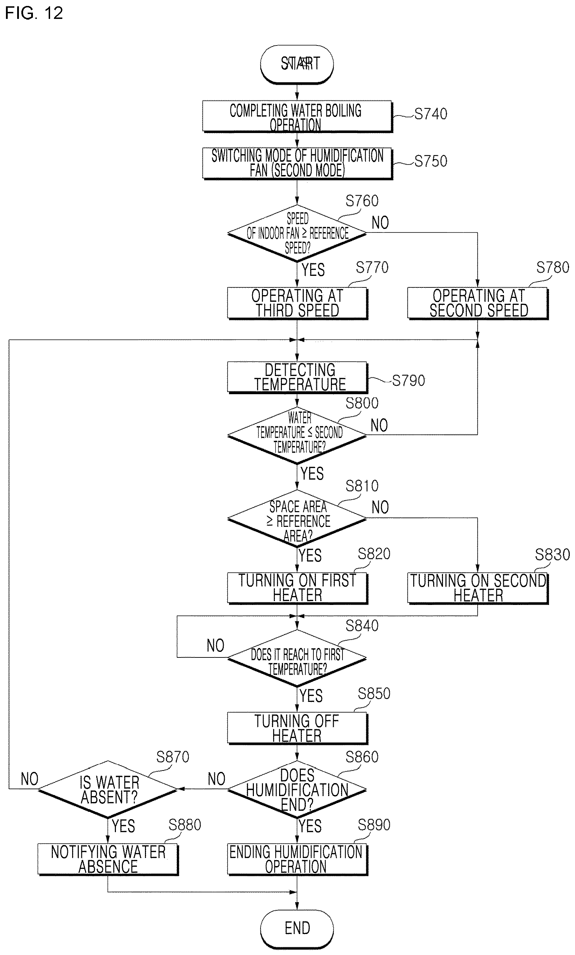

FIG. 12 is a flowchart of a method for performing a humidification operation by an air conditioner according to an embodiment of the present disclosure.

As shown in FIG. 12, after water boiling is completed (S740), as described above, a humidification module controller 3310 stops an operation (OFF) of a heater.

The humidification module controller 3310 does not stop a humidification fan 2500, but continuously operates the humidification fan 2500 by changing a rotational speed of the humidification fan. A mode of the humidification fan 2500 is switched from sterilization wind to humidification wind. The humidification wind rotates at a higher speed than that of the sterilization wind and the rotational speed thereof is set based on a rotational speed of an indoor fan 300.

When increasing the rotational speed, the humidification fan 2500 may not immediately change the rotational speed to a target rotational speed, but increases the speed step by step in a certain unit. For example, the humidification fan 2500 does not immediately increase the rotational speed from 1120 rpm of the sterilization wind to 2000 to 2500 rpm of the humidification wind, but increases the speed step by step in units of a predetermined time period. Air volume at a discharge outlet may be about 0.45 to 0.5 cmm.

For example, the humidification fan may increase the rotational speed by 50 rpm per minute. When the rotational speed of the humidification fan is rapidly changed, there is a problem in that the humidified air excessively flows into a water supply pipe, thereby increasing a temperature of the water supply pipe and water may flow backward. Therefore, the rotational speed of the humidification fan may be increased step by step.

Based on a current speed of the indoor fan being equal to or greater than a reference speed, the humidification module controller 3310 rotates the humidification fan at a third speed (S770). Based on the rotational speed of the indoor fan being less than the reference speed, the humidification module controller 3310 may rotate the humidification fan at a second speed that is faster than the first speed and slower than the third speed (S780).

The humidification module controller 3310 controls a temperature sensor to sense a temperature of water inside the steam generator while operating the humidification fan in a state in which the operation of the heater is stopped (S790).

The humidification module controller 3310 operates the heater of the steam generator based on the water temperature being reduced to a second temperature or less (S800). The first temperature described above may be set within a range of 95 to 100 Celsius degrees with respect to 100 Celsius degrees, which is the boiling point of water, and the second temperature may be set based on a temperature at which humidified air is not generated. The second temperature may be about 65 to70 Celsius degrees.

If a space is wide, the humidification module operates the first heater to perform a humidification operation, and if a space is narrow, the humidification operation operates the second heater to perform a humidification operation.

The humidification module controller 3310 may operate (ON) the first heater (S820) or operate the second heater (ON) (S830) according to a size of a space where an indoor unit is disposed. The first heater has a larger capacity than that of the second heater.

After the heater operation, the humidification module controller 3310 determines whether the water temperature of the steam generator reaches to a first temperature (S840). Based on the water temperature reaching the first temperature, an operation of the heater is stopped (OFF).

Until the humidification mode is released or the humidification operation is finished, the humidification module controller 3310 operates the humidification fan with the humidification wind and stops the heater based on the water temperature reaching to the first temperature and operates the heater again based on the water temperature reaching the second temperature that is lower than the first temperature. These operations are repeated (S790 to S860). Based on the above operations, the humidification module discharges humidified air.

Based on the humidification mode release or the end of humidification operation, the humidification module controller 3310 ends the humidification operation (S890).

Meanwhile, based on the insufficient water during the humidification operation (S870), the humidification module controller 3310 transmits a no-water notification to output a warning (S880). The humidification module controller 3310 may stop the humidification operation based on the water shortage. In this case, when water is supplied to the water tank after waiting for a predetermined time period, the humidification operation may restart.

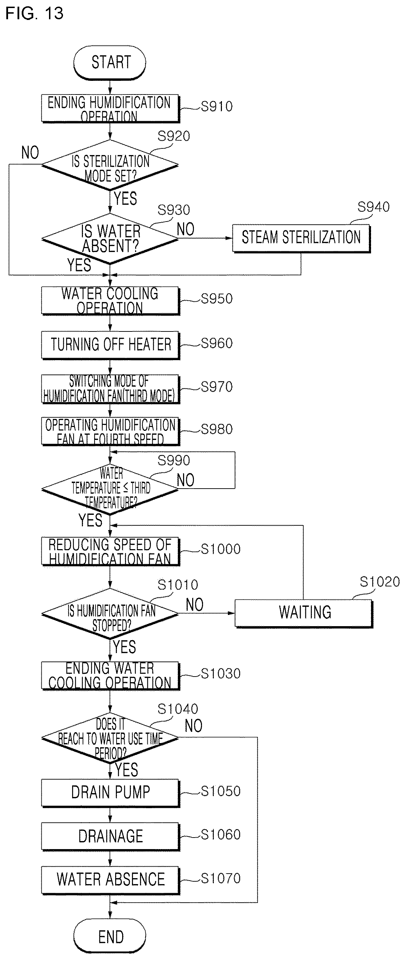

FIG. 13 is a flowchart of a method for performing a water cooling operation by an air conditioner according to an embodiment of the present disclosure.

As shown in FIG. 13, after a humidification operation is finished (S910), a humidification module controller 3310 determines whether a sterilization mode is set (S920), detects a level of water in a water tank, and determines whether water is insufficient or not (S930). The humidification module controller 3310 determines the water levels in the sterilization mode and when the sterilization mode is released. In the sterilization mode, based on sufficient water, the humidification module controller 3310 performs a steam sterilization operation (S940). In the steam sterilization operation, the humidification fan is operated with low-speed sterilization wind, and both the first heater and the second heater are operated to sterilize a steam guide and a discharge outlet.

After the humidification operation is finished, based on the sterilization mode being not set or when the steam sterilization is not performed due to insufficient water, the humidification module controller 3310 operates a water cooling operation (S950).

The water cooling operation is an operation of cooling the temperature of the water inside the steam generator heated by the humidification operation.

The humidification module controller 3310 stops the operation of the heater (S960) and switches a mode of a humidification fan from humidification wind to drying wind, which is a third mode (S970). The humidification fan 2500 rotates with the drying wind having maximum air volume and may operate at a fourth speed, which is higher than the third speed (S980).

As described above, the humidification fan increases the speed step by step in units of a predetermined time period. The drying wind cools the water, removes moisture generated due to the humidification operation, and is set to rotate at a higher speed than that of the humidification wind. For example, the rotational speed is set within the range of 3000 to 3300 rpm. Air volume at the discharge outlet may be about 0.6 to 0.7 cmm.

The humidification module controller 3310 detects the water temperature and determines whether the water temperature reaches a third temperature that is lower than the second temperature (S990). The third temperature is preferably set to be a temperature that does not damage a drain pump or a drain hose when mixed with the water in the water tank.

The humidification module controller 3310 reduces a speed of the humidification fan 2500 based on the water temperature inside the steam generator reaching the third temperature.

Until the operation of the humidification fan is stopped (S1010), the speed is reduced step by step in units of a predetermined time period (S1020 to S1010).

Based on the operation of the humidification fan being stopped, the humidification module controller 3310 terminates a water cooling operation (S1030).

The humidification module controller 3310 checks whether a water use time period is reached (S1040), and based on the water use time period being not reached, the humidification module controller 3310 maintains the water in the water tank.

Meanwhile, based on the water use time period being reached, the drain pump is operated (S1050) to drain all the water inside the water tank and the steam generator. After the water is drained, a no water notification is displayed (S1070).

The drain pump or the drain hose may be damaged when the temperature of water in the steam generator before the drainage is equal to or higher than the third temperature. Therefore, the humidification module controller 3310 performs drainage when the water temperature is equal to or less than the third temperature. In addition, when the water is drained, the water in the steam generator and the water in the water tank are mixed and are introduced into the drain pump. Therefore, water having a temperature lower than that of the water in the steam generator may flow to the drain pump.

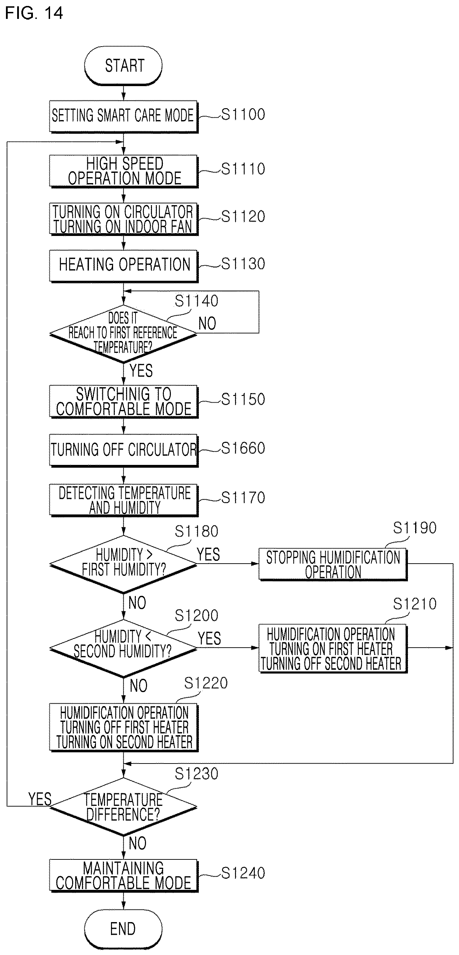

FIG. 14 is a flowchart of a method for automatically controlling a temperature and humidity by an air conditioner according to an embodiment of the present disclosure.

As shown in FIG. 14, even if a humidification mode is not set, a humidification operation may be performed during a cooling or heating operation of discharging heat-exchanged air. When a smart care mode is set, a controller controls an indoor unit to be operated in either a high speed mode or a comfortable mode and switches the mode according to the temperature and the humidity.

After the smart care mode is set (S1100), the controller controls a compressor and a fan by firstly setting the high speed mode (S1110).

The controller operates (ON) a circulator 400 and an indoor fan 300 (S1120) in the high speed mode. In this case, the circulator is a long-distance fan and the indoor fan is a short-distance fan.

In addition, the controller adjusts a valve and operates a compressor to perform a heating operation (S1130).

The controller controls a temperature sensor to measure a temperature of suctioned air and detect an indoor temperature, and based on the indoor temperature reaching a first reference temperature during the heating operation, the controller releases the high speed mode and switches to the comfortable mode (S1150).

Based on the setting of the comfortable mode, the controller stops an operation (OFF) of a circulator (S1160) and detects the temperature and humidity of an indoor space (S1170). The temperature sensor detects the indoor temperature and the humidity sensor detects relative humidity. The temperature sensor and the humidity sensor may be each disposed in a suction inlet, and in some cases, may each be disposed in a remote controller and the like. In addition, the temperature-humidity sensor may be configured as a device.

Based on the humidity being greater than first humidity, the controller stops the humidification operation that has been performed (S1190). If the humidification operation is in a stopped state, the stopped state is maintained.

Based on the humidity being equal to or lower than the first humidity and higher than the second humidity, the operation of the first heater is stopped and the second heater having a small capacity is operated to perform a humidification operation.

Based on the humidity being lower than the second humidity, the first heater is operated and the operation of the second heater is stopped to perform a humidification operation (S1210).

The controller transmits a control command to the humidification module controller, and when the humidification operation is in progress, checks an operation state of the heater and controls the heater to maintain the humidification operation, and when the humidification operation is not performed, controls the humidification operation to be performed.

As described above, the water boiling operation may be performed first prior to the humidification operation. During the humidification operation, the aforementioned water management, water boiling operation, humidification operation, steam sterilization operation, and water cooling operation may be performed. As the humidification operation may be repeatedly performed and stopped, the steam sterilization operation and the water cooling operation are preferably performed at a time point when all operations are finished.

In addition, the controller compares a desired temperature with an indoor temperature, and if a temperature difference from the desired temperature is less than a predetermined temperature, the controller maintains the comfortable mode (S1240).

Based on the temperature difference being equal to or higher than a predetermined temperature (S1230), the controller releases the comfortable mode and switches the mode to the high speed mode (S1110). The controller controls the fan in the high speed mode and maintains the high speed mode or switches the mode to the comfortable mode based on the first reference temperature being reached or being not reached to control the operation thereof based on the temperature and the humidity.

Therefore, the indoor unit of the air conditioner sets the smart care mode to prevent the frequent on and off and automatically switch the mode between the comfortable mode and the high speed mode without additional setting, thereby providing an indoor environment in which the predetermined temperature and humidity are maintained.

Embodiments of the present disclosure are described with reference to the accompanying drawings. The disclosure may, however, be embodied in many different manners and should not be construed as limited to the embodiments set forth herein. It is understood that a person having ordinary knowledge in the art to which the present disclosure pertains would implement this disclosure in other specific manners without changing the technical idea or necessary features of the present disclosure. Therefore, the disclosed embodiments are intended to be illustrative in all aspects, and not restrictive.

## Claims

1. An air conditioner, comprising:
a fan, including a short-air discharge distance fan assembly (300) and a long-air discharge distance fan assembly (400), for discharging air;
a cabinet assembly (100) defining a suction inlet (101) and a discharge outlet (301, 302);
a compressor ;
a water tank (2100) disposed within the cabinet assembly (100) and configured to store water;
a steam generator (2300) disposed within the cabinet assembly (100) and configured to receive the water stored in the water tank (2100),
wherein the steam generation (2300) converts the stored water into steam to produce humidified air;
a humidification fan (2500) coupled to the steam generator (2300) and for supplying filtered air to the steam generator (2300); and
a controller (3240, 3310) configured to control an operation of the air conditioner,
wherein, in a smart care mode, the controller (3240, 3310) is configured to control operation of the steam generator (2300) during a heating operation and controlling the compressor and the fan (300, 400) thereby discharging the humidified air together with heat-exchanged air to continuously adjust a temperature and humidity,
wherein, during a steam sterilization operation, the controller (3240, 3310) is configured to operate a heater of the steam generator to generate the humidified air, and operate the humidification fan (2500),
wherein a rotation speed of the humidification fan (2500) during the steam sterilization operation is lower than the rotation speed of the humidification fan (2500) in the smart care mode.

2. The air conditioner of claim 1, wherein, when the smart care mode is set, the controller (3240, 3310) is configured to execute one of a high speed mode or a comfortable mode, and to automatically switch between the high speed mode and the comfortable mode to adjust an indoor temperature and humidity.

3. The air conditioner of claim 2, wherein, when the high speed mode is set, the controller (3240, 3310) is configured to switch the high speed mode to the comfortable mode based on the indoor temperature reaching a desired temperature;
or
wherein the controller (3240, 3310) is configured to switch the comfortable mode to the high speed mode based on a temperature difference between the indoor temperature and a desired temperature being equal to or higher than a set temperature.

4. The air conditioner of claim 2, wherein, when the high speed mode is set, the controller (3240, 3310) is configured to operate both the short-distance fan assembly (300) configured to provide an indirect air flow to a user and to discharge air in a lateral direction of the cabinet assembly (100), and the long-distance fan assembly (400) configured to provide a direct air flow to the user and to discharge air in a forward direction of the cabinet assembly (100) to change the indoor temperature to a desired temperature.

5. The air conditioner of claim 4, wherein, when the comfortable mode is set, the controller (3240, 3310) is configured to stop an operation of the long-distance fan assembly (400), detect the indoor temperature and indoor humidity, and determine whether the steam generator (2300) performs a humidification operation.

6. The air conditioner of claim 5, wherein the controller (3240, 3310) is configured to stop the operation of the steam generator (2300) based on the indoor humidity being equal to or greater than a first humidity and to operate the steam generator (2300) to perform the humidification operation based on the indoor humidity being less than a second humidity that is lower than the first humidity;
and preferably
wherein the controller (3240, 3310) is configured to operate a heater having small capacity among a plurality of heaters of the steam generator (2300), based on the indoor humidity being within the range from the second humidity to the first humidity.

7. The air conditioner of claim 5, further comprising:
a humidification module (2000) including the steam generator (2300) and the humidification fan (2500),
wherein, when the humidification module (2000) performs the humidification operation according to a control command of the controller (3240, 3310), the humidification module (2000) is configured to:
perform a water boiling operation in which water inside the steam generator (2300) is heated to a predetermined temperature before the humidification module (2000) performs the humidification operation and then subsequently perform the humidification operation.

8. The air conditioner of claim 7, wherein, after the humidification module (2000) finishes the humidification operation, and when the heating operation is finished or the smart care mode is released, the humidification module (2000) is configured to perform the steam sterilization operation or a water cooling operation.

9. The air conditioner of claim 7, wherein during the water boiling operation, the steam generator (2300) is configured to operate any one of a first heater and a second heater of the steam generator (2300) based on operating frequency of the compressor and
wherein the humidification fan (2500) is operated with low-speed sterilization air flow;
or
wherein the humidification module (2000) is configured to stop the water boiling operation based on a temperature of the water stored inside the steam generator (2300) reaching a first temperature, or a specified time period having elapsed.

10. The air conditioner of claim 7, wherein the steam generator (2300) is configured to operate any one of a first heater and a second heater during the humidification operation, and
wherein the humidification fan (2500) is operated with humidification air flow, a rotational speed of the humidification fan (2500) being determined based on a rotational speed of the short-distance fan assembly (300);
and preferably
wherein the humidification module (2000) is configured to control the steam generator (2300) during the humidification operation to maintain the temperature of the water stored inside the steam generator (2300) within the range from a second temperature to a first temperature that is higher than the second temperature.

11. The air conditioner of claim 8, further comprising:
a steam guide ;
wherein, during the water cooling operation, the humidification module (2000) is configured to stop the operation of the steam generator (2300) and set the air flow of the humidification fan (2500) as drying air flow with maximum air volume to reduce the temperature of the water stored in the steam generator (2300) and to dry the steam guide (2400) and the discharge outlet (301, 302) through which the humidified air flows; and preferably
wherein the humidification module (2000) is configured to:
operate the humidification fan (2500) with the drying air flow during the water cooling operation until the water temperature reaches a third temperature that is lower than the second temperature, and based on the water temperature reaching the third temperature, reduce a rotational speed of the humidification fan (2500) until stopping the operation thereof.

12. The air conditioner of claim 8, wherein the humidification module (2000) is configured to operate all of a plurality of heaters of the steam generator (2300) and operate the humidification fan (2500) with low-speed sterilization air flow during the steam sterilization operation.

13. A method for controlling an indoor unit of an air conditioner, comprising:
setting a smart care mode including a high speed mode and a comfortable mode;
performing a heating operation in the high speed mode;
discharging heat-exchanged air and humidified air by operating both a short-air discharge distance fan assembly (300) and a long-air discharge distance fan assembly (400);
switching the high speed mode to the comfortable mode based on an indoor temperature reaching a desired temperature;
detecting the indoor temperature and indoor humidity while operating in the comfortable mode;
performing a humidification operation by operating a steam generator (2300) and a humidification fan (2500) to maintain the indoor humidity within a specified humidity range;
switching back to the high speed mode based on a temperature difference between the indoor temperature and the desired temperature reaching a predetermined value; and
performing a steam sterilization operation by operating a steam generator (2300) to generate a humidified air and operating the humidification fan (2500),
wherein a rotation speed of the humidification fan (2500) during the steam sterilization operation is lower than the rotation speed of the humidification fan (2500) during the humidification operation.

14. The method of claim 13, the comfortable mode further comprising:
stopping the humidification operation based on the indoor humidity being greater than the specified humidity range;
performing the humidification operation based on the indoor humidity being less than the specified humidity range; and
performing the humidification operation by changing capacity of a heater of the steam generator (2300) based on the indoor humidity being within specified humidity range.

15. The method of claim 13, further comprising:
performing a water boiling operation to increase a temperature of water stored in the steam generator (2300) before performing the humidification operation;
or
performing a steam sterilization operation or a water cooling operation after the humidification operation is finished.

## Patentansprüche

1. Klimaanlage, die aufweist:
einen Ventilator, der eine Nahluftabgabe-Ventilatoranordnung (300) und eine Fernluftabgabe-Ventilatoranordnung (400) aufweist, um Luft abzugeben;
eine Gehäuseanordnung (100), die einen Ansaugeinlass (101) und einen Abgabeauslass (301, 302) definiert;
einen Verdichter;
einen Wasserbehälter (2100), der in der Gehäuseanordnung (100) angeordnet und zum Speichern von Wasser konfiguriert ist;
einen Dampfgenerator (2300), der innerhalb der Gehäuseanordnung (100) angeordnet und konfiguriert ist, das in dem Wasserbehälter (2100) gespeicherte Wasser aufzunehmen,
wobei der Dampfgenerator (2300) das gespeicherte Wasser in Dampf umwandelt, um befeuchtete Luft zu erzeugen;
ein Befeuchtungsventilator (2500), der mit dem Dampfgenerator (2300) gekoppelt ist, um dem Dampfgenerator (2300) gefilterte Luft zuzuführen; und
eine Steuerung (3240, 3310), die konfiguriert ist, einen Betrieb der Klimaanlage zu steuern,
wobei in einem Smart-Care-Modus die Steuerung (3240, 3310) konfiguriert ist, den Betrieb des Dampfgenerators (2300) während eines Heizbetriebs zu steuern und den Verdichter und den Ventilator (300, 400) zu steuern, wodurch die befeuchtete Luft zusammen mit wärmegetauschter Luft abgegeben wird, um eine Temperatur und Feuchtigkeit kontinuierlich einzustellen,
wobei während eines Dampfsterilisationsbetriebs die Steuerung (3240, 3310) konfiguriert ist, eine Heizung des Dampfgenerators zu betreiben, um die befeuchtete Luft zu erzeugen, und den Befeuchtungsventilator (2500) zu betreiben,
wobei eine Rotationsgeschwindigkeit des Befeuchtungsventilators (2500) während des Dampfsterilisationsbetriebs niedriger ist als die Rotationsgeschwindigkeit des Befeuchtungsventilators (2500) im Smart-Care-Modus.

2. Klimaanlage nach Anspruch 1, wobei, wenn der Smart-Care-Modus eingestellt ist, die Steuerung (3240, 3310) konfiguriert ist, einen eines Hochgeschwindigkeitsmodus oder eines Komfortmodus auszuführen und automatisch zwischen dem Hochgeschwindigkeitsmodus und dem Komfortmodus umzuschalten, um eine Innentemperatur und Feuchtigkeit einzustellen.

3. Klimaanlage nach Anspruch 2, wobei, wenn der Hochgeschwindigkeitsmodus eingestellt ist, die Steuerung (3240, 3310) konfiguriert ist, den Hochgeschwindigkeitsmodus auf den Komfortmodus basierend darauf umzuschalten, dass die Innentemperatur eine gewünschte Temperatur erreicht;
oder
wobei die Steuerung (3240, 3310) konfiguriert ist, den Komfortmodus auf den Hochgeschwindigkeitsmodus basierend darauf umzuschalten, dass eine Temperaturdifferenz zwischen der Innentemperatur und einer gewünschten Temperatur gleich oder höher als eine eingestellte Temperatur ist.

4. Klimaanlage nach Anspruch 2, wobei, wenn der Hochgeschwindigkeitsmodus eingestellt ist, die Steuerung (3240, 3310) konfiguriert ist, sowohl die Nahluftabgabe-Ventilatoranordnung (300), die konfiguriert ist, einen indirekten Luftstrom für einen Benutzer bereitzustellen und Luft in einer seitlichen Richtung der Gehäuseanordnung (100) abzugeben, als auch die Fernluftabgabe-Ventilatoranordnung (400) zu betreiben, die konfiguriert ist, einen direkten Luftstrom für den Benutzer bereitzustellen und Luft in einer Vorwärtsrichtung der Gehäuseanordnung (100) abzugeben, um die Innentemperatur auf eine gewünschte Temperatur zu ändern.

5. Klimaanlage nach Anspruch 4, wobei die Steuerung (3240, 3310) konfiguriert ist, wenn der Komfortmodus eingestellt ist, einen Betrieb der Fernluftabgabe-Ventilatoranordnung (400) zu stoppen, die Innentemperatur und die Innenfeuchtigkeit zu erfassen und zu bestimmen, ob der Dampfgenerator (2300) einen Befeuchtungsbetrieb durchführt.

6. Klimaanlage nach Anspruch 5, wobei die Steuerung (3240, 3310) konfiguriert ist, den Betrieb des Dampfgenerators (2300) basierend darauf zu stoppen, dass die Innenfeuchtigkeit gleich oder größer als eine erste Feuchtigkeit ist, und den Dampfgenerator (2300) zu betreiben, den Befeuchtungsbetrieb basierend darauf durchzuführen, dass die Innenfeuchtigkeit geringer als eine zweite Feuchtigkeit ist, die geringer als die erste Feuchtigkeit ist;
und vorzugsweise
wobei die Steuerung (3240, 3310) konfiguriert ist, eine Heizung mit kleiner Leistung unter mehreren Heizungen des Dampfgenerators (2300) basierend darauf zu betreiben, dass die Innenfeuchtigkeit innerhalb des Bereichs von der zweiten Feuchtigkeit bis zur ersten Feuchtigkeit liegt.

7. Klimaanlage nach Anspruch 5, die ferner aufweist:
ein Befeuchtungsmodul (2000), das den Dampfgenerator (2300) und den Befeuchtungsventilator (2500) aufweist,
wobei, wenn das Befeuchtungsmodul (2000) den Befeuchtungsbetrieb nach einem Steuerbefehl der Steuerung (3240, 3310) durchführt, das Befeuchtungsmodul (2000) konfiguriert ist, um:
einen Wasserkochbetrieb durchzuführen, bei dem Wasser im Inneren des Dampfgenerators (2300) auf eine vorbestimmte Temperatur erhitzt wird, bevor das Befeuchtungsmodul (2000) den Befeuchtungsbetrieb durchführt, und dann anschließend den Befeuchtungsbetrieb durchzuführen.

8. Klimaanlage nach Anspruch 7, wobei nachdem das Befeuchtungsmodul (2000) den Befeuchtungsbetrieb beendet und wenn der Heizbetrieb beendet ist oder der Smart-Care-Modus freigegeben ist, das Befeuchtungsmodul (2000) konfiguriert ist, den Dampfsterilisationsbetrieb oder einen Wasserkühlbetrieb durchzuführen.

9. Klimaanlage nach Anspruch 7, wobei während des Wasserkochbetriebs der Dampfgenerator (2300) konfiguriert ist, eine einer ersten Heizung und einer zweiten Heizung des Dampfgenerators (2300) basierend auf der Betriebsfrequenz des Verdichters zu betreiben, und wobei das Befeuchtungsventilator (2500) mit einem Sterilisationsluftstrom niedriger Geschwindigkeit betrieben wird;
oder
wobei das Befeuchtungsmodul (2000) konfiguriert ist, den Wasserkochbetrieb basierend darauf zu stoppen, dass eine Temperatur des im Dampfgenerator (2300) gespeicherten Wassers eine erste Temperatur erreicht oder eine spezifizierte Zeitspanne verstrichen ist.

10. Klimaanlage nach Anspruch 7, wobei der Dampfgenerator (2300) konfiguriert ist, während des Befeuchtungsbetriebs eine einer ersten Heizung oder einer zweiten Heizung zu betreiben, und
wobei der Befeuchtungsventilator (2500) mit einem Befeuchtungsluftstrom betrieben wird,
wobei eine Rotationsgeschwindigkeit des Befeuchtungsventilators (2500) basierend auf einer Rotationsgeschwindigkeit der Nahluftabgabe-Ventilatoranordnung (300) bestimmt wird;
und vorzugsweise
wobei das Befeuchtungsmodul (2000) konfiguriert ist, den Dampfgenerator (2300) während des Befeuchtungsbetriebs zu steuern, die Temperatur des in dem Dampfgenerator (2300) gespeicherten Wassers in dem Bereich zwischen einer zweiten Temperatur und einer ersten Temperatur zu halten, die höher als die zweite Temperatur ist.

11. Klimaanlage nach Anspruch 8, die ferner aufweist:
eine Dampfführung;
wobei während des Wasserkühlungsbetriebs das Befeuchtungsmodul (2000) konfiguriert ist, den Betrieb des Dampfgenerators (2300) zu stoppen und den Luftstrom des Befeuchtungsventilators (2500) als Trocknungsluftstrom mit maximalem Luftvolumen einzustellen, um die Temperatur des im Dampfgenerator (2300) gespeicherten Wassers zu verringern und die Dampfführung (2400) und den Auslass (301, 302), durch den die befeuchtete Luft strömt, zu trocknen; und vorzugsweise
wobei das Befeuchtungsmodul (2000) konfiguriert ist, um:
das Befeuchtungsventilator (2500) mit dem Trocknungsluftstrom während des Wasserkühlungsbetriebs zu betreiben, bis die Wassertemperatur eine dritte Temperatur erreicht, die niedriger als die zweite Temperatur ist, und basierend darauf, dass die Wassertemperatur die dritte Temperatur erreicht, eine Rotationsgeschwindigkeit des Befeuchtungsventilators (2500) zu reduzieren, bis dessen Betrieb gestoppt wird.

12. Klimaanlage nach Anspruch 8, wobei das Befeuchtungsmodul (2000) konfiguriert ist, alle von mehreren Heizungen des Dampfgenerators (2300) zu betreiben und den Befeuchtungsventilator (2500) während des Dampfsterilisationsbetriebs mit einem Sterilisationsluftstrom niedriger Geschwindigkeit zu betreiben.

13. Verfahren zur Steuerung einer Inneneinheit einer Klimaanlage, das aufweist Einstellen eines Smart-Care-Modus, der einen Hochgeschwindigkeitsmodus und einen Komfortmodus aufweist;
Durchführen eines Heizbetriebs im Hochgeschwindigkeitsmodus;
Abgeben von wärmegetauschter Luft und befeuchteter Luft durch Betreiben sowohl einer Nahluftabgabe-Ventilatoranordnung (300) als auch einer Fernluftabgabe-Ventilatoranordnung (400);
Umschalten des Hochgeschwindigkeitsmodus auf den Komfortmodus basierend darauf, dass eine Innentemperatur eine gewünschte Temperatur erreicht;
Erfassen der Innentemperatur und der Innenfeuchtigkeit während des Betriebs im Komfortmodus;
Durchführen eines Befeuchtungsbetriebs durch Betreiben eines Dampfgenerators (2300) und eines Befeuchtungsventilators (2500), um die Innenfeuchtigkeit innerhalb eines bestimmten Feuchtigkeitsbereichs zu halten;
Zurückschalten in den Hochgeschwindigkeitsmodus basierend darauf, dass eine Temperaturdifferenz zwischen der Innentemperatur und der gewünschten Temperatur einen vorbestimmten Wert erreicht; und
Durchführen eines Dampfsterilisationsbetriebs durch Betreiben eines Dampfgenerators (2300) zum Erzeugen befeuchteter Luft und Betreiben des Befeuchtungsventilators (2500),
wobei eine Rotationsgeschwindigkeit des Befeuchtungsventilators (2500) während des Dampfsterilisationsbetriebs niedriger ist als die Rotationsgeschwindigkeit des Befeuchtungsventilators (2500) während des Befeuchtungsbetriebs.

14. Verfahren nach Anspruch 13, wobei der Komfortmodus ferner aufweist:
Stoppen des Befeuchtungsbetriebs basierend darauf, dass sie Innenfeuchtigkeit größer als der spezifizierte Feuchtigkeitsbereich ist;
Durchführen des Befeuchtungsbetriebs basierend darauf, dass die Raumfeuchtigkeit kleiner als der spezifizierte Feuchtigkeitsbereich ist; und
Durchführen des Befeuchtungsbetriebs durch Ändern der Leistung einer Heizung des Dampfgenerators (2300) basierend darauf, dass die Innenfeuchtigkeit innerhalb des spezifizierten Feuchtigkeitsbereichs liegt.

15. Verfahren nach Anspruch 13, das ferner aufweist:
Durchführen eines Wasserkochbetriebs, um eine Temperatur des in dem Dampfgenerator (2300) gespeicherten Wassers vor dem Durchführen des Befeuchtungsbetriebs zu erhöhen;
oder
Durchführen eines Dampfsterilisationsbetriebs oder eines Wasserkühlbetriebs, nachdem der Befeuchtungsbetrieb beendet ist.

## Revendications

1. Climatiseur, comprenant :
un ventilateur, comportant un ensemble de ventilateur à faible distance de refoulement d'air (300) et un ensemble de ventilateur à longue distance de refoulement d'air (400), pour refouler de l'air ;
un ensemble de carrosserie (100) définissant une entrée d'aspiration (101) et une sortie de refoulement (301, 302) ;
un compresseur ;
un réservoir d'eau (2100) disposé à l'intérieur de la carrosserie (100) et prévu pour stocker de l'eau ;
un générateur de vapeur (2300) disposé à l'intérieur de l'ensemble de carrosserie (100) et prévu pour recevoir l'eau stockée dans le réservoir d'eau (2100),
où le générateur de vapeur (2300) transforme en vapeur l'eau stockée pour produire de l'air humidifié ;
un ventilateur d'humidification (2500) raccordé au générateur de vapeur (2300) et destiné à refouler de l'air filtré vers le générateur de vapeur (2300) ; et
un contrôleur (3240, 3310) prévu pour commander le fonctionnement du climatiseur,
où, dans un mode de protection intelligent, le contrôleur (3240, 3310) est prévu pour commander le fonctionnement du générateur de vapeur (2300) pendant un processus de chauffage et pour commander le compresseur et le ventilateur (300, 400), refoulant ainsi l'air humidifié avec l'air soumis à échange de chaleur afin d'ajuster la température et l'humidité de manière continue,
où, lors d'un processus de stérilisation par vapeur, le contrôleur (3240, 3310) est prévu pour faire fonctionner un élément chauffant du générateur de vapeur afin de générer de l'air humidifié, et pour activer le ventilateur d'humidification (2500),
où la vitesse de rotation du ventilateur d'humidification (2500) pendant le processus de stérilisation par vapeur est inférieure à la vitesse de rotation du ventilateur d'humidification (2500) en mode de protection intelligent.

2. Climatiseur selon la revendication 1, où, si le mode de protection intelligent est réglé, le contrôleur (3240, 3310) est prévu pour exécuter soit un mode haute vitesse soit un mode confort, et pour basculer automatiquement entre le mode haute vitesse et le mode confort afin d'ajuster une température et une humidité intérieures.

3. Climatiseur selon la revendication 2, où, si le mode haute vitesse est réglé, le contrôleur (3240, 3310) est prévu pour passer du mode haute vitesse au mode confort si la température intérieure atteint une température souhaitée ;
ou
où le contrôleur (3240, 3310) est prévu pour passer du mode confort au mode haute vitesse si une différence de température entre la température intérieure et une température souhaitée est égale ou supérieure à une température définie.

4. Climatiseur selon la revendication 2, où, si le mode haute vitesse est réglé, le contrôleur (3240, 3310) est prévu pour activer à la fois l'ensemble de ventilateur à faible distance (300) prévu pour fournir un flux d'air indirect à un utilisateur et pour refouler de l'air dans une direction latérale de l'ensemble de carrosserie (100), et l'ensemble de ventilateur à longue distance (400) prévu pour fournir un flux d'air direct à l'utilisateur et pour refouler de l'air vers l'avant de l'ensemble de carrosserie (100) afin de passer la température intérieure à une température souhaitée.

5. Climatiseur selon la revendication 4, où, si le mode confort est réglé, le contrôleur (3240, 3310) est prévu pour arrêter le fonctionnement de l'ensemble de ventilateur à longue distance (400), détecter la température intérieure et l'humidité intérieure, et déterminer si le générateur de vapeur (2300) exécute un processus d'humidification.

6. Climatiseur selon la revendication 5, où le contrôleur (3240, 3310) est prévu pour arrêter le fonctionnement du générateur de vapeur (2300) si l'humidité intérieure est égale ou supérieure à une première humidité et pour activer le générateur de vapeur (2300) afin d'exécuter le processus d'humidification si l'humidité intérieure est inférieure à une deuxième humidité inférieure à la première humidité ;
et où, de préférence,
le contrôleur (3240, 3310) est prévu pour activer un élément chauffant de faible capacité parmi une pluralité d'éléments chauffants du générateur de vapeur (2300), si l'humidité intérieure est comprise entre la deuxième et la première humidité.

7. Climatiseur selon la revendication 5, comprenant en outre :
un module d'humidification (2000) comportant le générateur de vapeur (2300) et le ventilateur d'humidification (2500),
où, si le module d'humidification (2000) exécute le processus d'humidification en fonction d'une commande du contrôleur (3240, 3310), le module d'humidification (2000) est prévu pour :
exécuter un processus d'ébullition de l'eau où l'eau à l'intérieur du générateur de vapeur (2300) est chauffée à une température prédéterminée avant exécution par le module d'humidification (2000) du processus d'humidification, suivi de l'exécution du processus d'humidification.

8. Climatiseur selon la revendication 7, où, à l'issue du processus d'humidification par le module d'humidification (2000), et à l'issue du processus de chauffage ou lors de l'activation du mode de protection intelligent, le module d'humidification (2000) est prévu pour exécuter le processus de stérilisation par vapeur ou un processus de refroidissement par eau.

9. Climatiseur selon la revendication 7, où, pendant le processus d'ébullition de l'eau, le générateur de vapeur (2300) est prévu pour activer un premier élément chauffant ou un deuxième élément chauffant du générateur de vapeur (2300) sur la base de la fréquence de fonctionnement du compresseur, et
où le ventilateur d'humidification (2500) est activé avec un flux d'air de stérilisation à faible vitesse ;
ou
où le module d'humidification (2000) est prévu pour arrêter le processus d'ébullition de l'eau si la température de l'eau stockée dans le générateur de vapeur (2300) atteint une première température, ou après écoulement d'une durée spécifiée.

10. Climatiseur selon la revendication 7, où le générateur de vapeur (2300) est prévu pour activer un premier élément chauffant ou un deuxième élément chauffant pendant le processus d'humidification, et.
où le ventilateur d'humidification (2500) est activé avec un flux d'air d'humidification, une vitesse de rotation du ventilateur d'humidification (2500) étant déterminée sur la base d'une vitesse de rotation de l'ensemble de ventilateur à faible distance (300) ;
et où, de préférence,
le module d'humidification (2000) est prévu pour commander le générateur de vapeur (2300) pendant le processus d'humidification afin de maintenir la température de l'eau stockée dans le générateur de vapeur (2300) dans une plage allant d'une deuxième température à une première température supérieure à la deuxième température.

11. Climatiseur selon la revendication 8, comprenant en outre:
un guidage de vapeur ;
où, pendant le processus de refroidissement d'eau, le module d'humidification (2000) est prévu pour arrêter le fonctionnement du générateur de vapeur (2300) et régler le débit d'air du ventilateur d'humidification (2500) en tant que débit d'air de séchage avec un volume d'air maximal, afin de réduire la température de l'eau stockée dans le générateur de vapeur (2300) et sécher le guidage de vapeur (2400) et la sortie de refoulement (301, 302) par laquelle l'air humidifié s'écoule ; et où, de préférence,
le module d'humidification (2000) est prévu pour :
activer le ventilateur d'humidification (2500) avec le flux d'air de séchage pendant le processus de refroidissement d'eau jusqu'à ce que la température de l'eau atteigne une troisième température inférieure à la deuxième température, et, si la température de l'eau atteint la troisième température, réduire une vitesse de rotation du ventilateur d'humidification (2500) jusqu'à l'arrêt de son fonctionnement.

12. Climatiseur selon la revendication 8, où le module d'humidification (2000) est prévu pour activer tous les éléments chauffants d'une pluralité d'éléments chauffants du générateur de vapeur (2300), et activer le ventilateur d'humidification (2500) avec un flux d'air de stérilisation à faible vitesse pendant le processus de stérilisation par vapeur.

13. Procédé de commande d'une unité intérieure d'un climatiseur, comprenant :
le réglage d'un mode de protection intelligent comprenant un mode haute vitesse et un mode confort ;
l'exécution d'un processus de chauffage en mode haute vitesse ;
le refoulement de l'air soumis à échange de chaleur et de l'air humidifié par activation d'un ensemble de ventilateur à faible distance de refoulement d'air (300) et d'un ensemble de ventilateur à longue distance de refoulement d'air (400) ;
la commutation du mode haute vitesse vers le mode confort si la température intérieure atteint une température souhaitée ;
la détection de la température et de l'humidité intérieures pendant le fonctionnement en mode confort ;
l'exécution d'un processus d'humidification par activation d'un générateur de vapeur (2300) et d'un ventilateur d'humidification (2500) pour maintenir l'humidité intérieure dans une plage d'humidité spécifiée ;
le retour au mode haute vitesse si la différence de température entre la température intérieure et la température souhaitée atteint une valeur prédéterminée ; et
l'exécution d'un processus de stérilisation par vapeur par activation d'un générateur de vapeur (2300) pour générer de l'air humidifié et par activation du ventilateur d'humidification (2500),
où la vitesse de rotation du ventilateur d'humidification (2500) pendant le processus de stérilisation par vapeur est inférieure à la vitesse de rotation du ventilateur d'humidification (2500) pendant le processus d'humidification.

14. Procédé selon la revendication 13, où le mode confort comprend en outre :
l'arrêt du processus d'humidification si l'humidité intérieure est supérieure à la plage d'humidité spécifiée ;
l'exécution du processus d'humidification si l'humidité intérieure est inférieure à la plage d'humidité spécifiée ; et
l'exécution du processus d'humidification par modification de la capacité d'un élément chauffant du générateur de vapeur (2300) si l'humidité intérieure est comprise dans la plage d'humidité spécifiée.

15. Procédé selon la revendication 13, comprenant en outre :
l'exécution d'un processus d'ébullition de l'eau pour augmenter la température de l'eau stockée dans le générateur de vapeur (2300) avant exécution du processus d'humidification ;
ou
l'exécution d'un processus de stérilisation par vapeur ou d'un processus de refroidissement d'eau à l'issue du processus d'humidification.
